# EUROPÄISCHE PATENTSCHRIFT

(11) **EP 0 315 782 B1**
(45) Veröffentlichungstag und Bekanntmachung des Hinweises auf die Patenterteilung: **28.09.1994**
(21) Anmeldenummer: 88116739.9
(22) Anmeldetag: 10.10.1988
(51) Int. Cl.: C12N 1/38, C12N 5/00, C12N 9/64, C12N 15/58

(54) **Verfahren zur Herstellung von Proteinen**
Method for the production of proteins
Méthode de production de protéines

(30) Priorität: 13.10.1987 DE 3734632; 13.11.1987 DE 3738649; 20.01.1988 DE 3801562
(43) Veröffentlichungstag der Anmeldung: 17.05.1989
(73) Patentinhaber: Dr. Karl Thomae GmbH, D-88397 Biberach (DE)
(72) Erfinder: Stecher-Schilling, Andrea, Dr. Dipl.-Biologin., D-7950 Biberach 1 (DE); Werner, Rolf-Günter, Dr. Dipl.-Mikrobiologe., D-7950 Biberach 1 (DE); Werz, William, Dr. Dipl.-Biologe., D-7951 Warthausen (DE); Zähner, Hans, Prof.Dr., D-7400 Tübingen (DE); Zeeck, Axel, Prof.Dr. Dipl.-Chem., D-3400 Göttingen (DE)

(56) Entgegenhaltungen:
- EP-A- 0 163 751
- EP-A- 0 239 292
- CHEMICAL ABSTRACTS, Band 109, Nr. 9, August 1988, Seite 561, Ref. Nr71883v; Columbus. Ohio, US; R.G. WERNER: "Biotechnical production of pharmaceuticals with special consideration of plasminogen activator"
- CHEMICAL ABSTRACTS, Band 105, Nr. 17, Oktober 1986, Seiten 569-570 Ref.Nr. 151573b; Columbus Ohio , US & JP-61 119 189
- BIOCHEM. J., Band 247, Nr. 3, 1987, Seiten 605-612; London GB T. KOOISTRA et al.: "Butyrate stimulates tissue-type plasminogen-activator synthesis in cultured human endothelial cells"
- NUCLEIC ACID RESEARCH, Band 11, Nr. 21, 1983, Seiten 7631-7648; IRL Press Ltd., Oxford, G.B., C.M. GORMAN et al.: "Expression of recombinant plasmids in mammalian cells is enhanced by sodium butyrate"
- CHEMICAL ABSTRACTS, Band 103, Nr. 5, August 1985, Rf.Nr. 3401u; Columbus, Ohio, US, N. ICHIKAWA et al.: "Production, purification and characterization of the plasminogen activator in teratocarcinoma stem cells induced with sodium butyrate" & BIKEN J., 1984, 27(4), 143-51.

## Beschreibung

Plasminogenaktivatoren sind eine Klasse von Serinproteasen, die das Proenzym Plasminogen durch Spaltung der Peptidbindung zwischen Arg-560 und Val-561 zum aktiven Enzym Plasmin aktivieren. Plasmin seinerseits ist die letzte Stufe des Fibrinolysesystems der Blutbahn, das das Fibringerüst eines Thrombus in lösliche Peptide spaltet.

Bei pathogenen Störungen, wie Koronarerkrankungen, werden Blutgerinnsel nicht schnell genug aufgelöst, um eine ausreichende Versorgung des Gewebes mit Sauerstoff zu gewährleisten. Die Folge des Verschlusses der Koronararterien durch ein Blutgerinnsel ist ein Infarkt, wobei durch Sauerstoffunterversorgung des Herzmuskels die Nekrose des betroffenen Gewebes erfolgt.

Eine rasche Rekanalisation der Gefäße kann die Durchblutung des Herzmuskels in kürzester Zeit sicherstellen und so das Absterben ganzer Muskelpartien des Herzens verhindern.

Bei der Therapie werden bislang Streptokinase und Urokinase eingesetzt. Eigenschaften von t-PA bieten jedoch einige Vorteile gegenüber jenen bekannten Plasminogenaktivatoren: die fibrinspezifische lokale Lyse, keine systemischen lytischen Effekte durch Fibrinogenabbau, hohe Reperfusionsraten und keine Antikörperbildung.

t-PA ist ein Glykoprotein mit einem Molekulargewicht von ca. 65.000 Dalton, das als einkettiges und zweikettiges Enzym vorkommt und aus 527 Aminosäuren zusammengesetzt ist.

Die Affinität von t-PA für Plasminogen ist in Gegenwart von Fibrin um ein 100-faches größer als in dessen Abwesenheit.

Diese hohe Affinität von t-PA für Plasminogen in Gegenwart von Fibrin sichert die effektive Aktivierung, ohne freies Plasminogen in der Peripherie zu aktivieren.

Humanes t-PA wurde zuerst aus Uteri in reiner Form gewonnen. In anderen Geweben und Zellen, z.B. in endothelialen Zellen der Blutgefäße, konnte gleichfalls t-PA nachgewiesen werden. Die gebildeten t-PA-Mengen sind jedoch so gering, daß ihre kommerzielle Gewinnung unmöglich ist.

Eine weitere natürliche Quelle für t-PA ist die Zellkultur. Mehrere Zell-Linien wurden auf eine mögliche t-PA-Produktion untersucht (Gronow M. et al., Trends in Biotechnology 1: 26-29, 1983). Aus einer humanen Zell-Linie, den Bowes-Melanoma-Zellen, konnten größere Mengen an t-PA für begrenzte klinische Studien sowie zur Aufklärung der Struktur gewonnen werden (Wallen et al., European Journal of Biochemistry 132: 681-686, 1983).

Für die Großproduktion von t-PA zur breiten klinischen Anwendung sind diese Ausbeuten allerdings zu gering. Erst die Gentechnik machte es möglich, größere Mengen rekombinantes t-PA für den therapeutischen Einsatz herzustellen.

1983 wurde das t-PA kloniert und die Aminosäuresequenz ermittelt (Pennica, Nature 301: 214-221, 1983).

Versuche, um t-PA aus E.coli, in dem die genetischen Informationen für t-PA aus Melanoma-Zellen integriert wurden, zu erhalten, waren nicht erfolgreich, da das resultierende Protein nicht glykosyliert ist und damit dem nativen t-PA nicht entspricht.

Aus diesem Grund haben verschiedene Forschungsgruppen für die Produktion von humanem rt-PA permanente Säugetier-Zell-Linien von verschiedenen Quellen ausgesucht. Einige von diesen Zell-Linien sind humane Zell-Linien mit angeblich verbesserter Ausbeute von t-PA. Eine andere Zell-Linie ist die chinesische Hamsterovarzelle.

In der Europäischen Offenlegungsschrift 0,093,619 wird die Herstellung von t-PA von transformierten chinesischen Hamsterovarzellen (CHO-Zellen) beschrieben. Das resultierende t-PA (rt-PA) unterscheidet sich in keiner Weise vom natürlich vorkommenden t-PA.

In mehreren Publikationen werden Mutanten der t-PA und deren Herstellung beschrieben, z.B. in EP-A-0,241,208, 0,241,209, 0,241,210, 0,240,334, 0,234,051, 0,233,013, 0,231,624, 0,225,286, 0,213,794, 0,201,153, 0,199,574, 0,196,920 und DE-A-3,708,681.

In der vorliegenden Anmeldung werden alle t-PA-Derivate Mutanten genannt, gleichgültig, ob sie eine oder mehrere Aminosäuren zusätzlich enthalten, sich von der Aminosäure in der gleichen Stellung im in der Natur vorkommenden t-PA unterscheiden oder worin eine oder mehrere Aminosäuren, die im natürlichen t-PA vorkommen, in diesen "Mutanten" nicht enthalten sind. Ein t-PA, worin viele Aminosäurereste des natürlich vorkommenden t-PA abwesend sind, wie z.B. in EP-A-0,196,920, wird manchmal "degraded species" genannt.

In der EP-A-0,199,574 wird z.B. eine rt-PA Mutante beschrieben, die in der Stellung 270 bis 279 Aminosäuren besitzt, die heterolog zu der Aminosäuresequenz im natürlichen t-PA ist.

In der DE-A-3,708,681 werden von der Anmelderin einige rt-PA's beschrieben, die in Stellung 117 eine Aminosäure besitzen, die sich von der Aminosäure in der entsprechenden Stellung beim natürlichen t-PA unterscheiden, wobei das mutierte rt-PA im Gegensatz zum natürlichen t-PA in dieser Stellung nicht glykosyliert ist.

In vielen Publikationen wird der Einfluß von verschiedenen Substanzen auf die Produktivität von Zellkulturen für die Synthese von Proteinen beschrieben.

Bei verschiedenen Zellsystemen wird von einem Zusammenhang zwischen intrazellulärem c-AMP-Spiegel und der t-PA-Synthese berichtet (siehe z.B. Kooistra et al. in Thrombosis and Haemostasis, 54(1): 192, Abstract P 1133), daß Dibutyryl c-AMP die Produktion von t-PA in humanen endothelialen Zellen um das 2- bis 3-fache erhöht, aber keinen Einfluß auf die Synthese von t-PA in Bowes-Melanoma-Zellen aufweist. In Nierenzellen wurde die t-PA-Synthese durch Phosphodiesterase-Inhibitoren gesteigert (Journal of Cell Biology 91: 195-200, 1981). Andererseits hemmt c-AMP die t-PA-Synthese in Makrophagen (Vassalli et al., Cells, 8: 271-281, 1976) und ist indifferent in Teratocarcinoma-Zellen (Nishimune et al., Experimental Cell Research 146: 439-444, 1983).

Buttersäure induziert die Synthese von t-PA in Teratocarcinomia-Zellen (Nishimune - siehe oben), zeigt aber in renalen Tumorzellen eine inhibitorische Wirkung auf (Nelson et al. Proc. Am. Ass. for Cancer Research, 26:35, 1985), und von einer indifferenten Wirkung in embryonalen Lungenzellen wurde von Brouty-Boye et al., Biotechnology 12: 10, 1984, berichtet.

In humanen Endothelzellen kann die t-PA-Synthese durch Thrombin (Levin et al., Thombosis and Haemostasis, 56(2): 115-119, 1986), Alkohol (Laug, Jama, 250(6): 772-776, 1983 B), Vitamin C und Vitamin A (Inada et al., Biochemical and Biophysical Communications 130(1): 182-187, 1985) stimuliert werden. Die t-PA-Sekretion in bovinen Endothelzellen wird jedoch durch Thrombin (Loskutoff, Journal of Clinical Investigations, 64: 329-332, 1979) und Endotoxin (Crutchley et al., Journal of Biological Chemistry, 261(1): 154-159, 1986) gehemmt.

Die t-PA-Sekretion aus dem Schwein-Endothel wird durch Heparin vermittelt (Marchwardt et al., Thombosis Research, 8: 217-223, 1976), bei den humanen embryonalen Lungenzellen hat Heparin allerdings einen minimalen Effekt.

Die t-PA-Synthese wurde weiterhin durch Concanavalin A in embryonalen Lungenzellen (Brouty-Boye - siehe oben) und Epithelzellen (Electricwala et al., Thrombosis and Haemostasis, 53: 200-203, 1985), 5-Azacytidin in Epithelzellen (Electricwala - siehe oben) und Melanoma-Zellen (Silagi et al., Biological Medicine, 57: 418, 1984), Tizabrine in Melanoma-Zellen (Roba et al., Thombosis and Haemostasis 50(1): 83, 1983) und Aphidicolin in Hepatoma-Zellen (Orfanoudakis et al., Biological Chemistry, Hoppe-Seyler, 336(9): 832, 1985) positiv beeinflußt.

In der Europäischen Offenlegungsschrift 0,219,270 wird beschrieben, daß eine Kombination von Heparin und Endothelialzellwachstumsfaktor (ECGF) die Produktion von t-PA und einkettigem Urokinaseplasminogenaktivator aus normalen humanen diploiden Lungenfibroblastzellen in serumfreiem Medium erhöht.

Über den Einfluß von Buttersäure auf chinesische Hamsterovarzellen wurde von Storrie et al., Journal of Cell Physiology, 94: 69-76, 1978, und Wright, Experimental Cell Research, 78: 456-460, 1978, berichtet. Jene Untersuchungen wurden allerdings mit nicht transformierten Zell-Linien durchgeführt und die Ergebnisse wurden im Großen und Ganzen auf die Änderungen der Morphologie und Wachstumsrate der Zellen gerichtet.

Außerdem wird in der EP-A-0,239,292 die Verwendung von Natriumbutyrat zur Steigerung der Proteinproduktion bei der Kultivierung von gentechnologisch veränderten Zellen oder von Hybridomen beschrieben, wobei nur die Verwendung von Natriumbutyrat bei der Antikörper-Produktion expressis verbis beschrieben wird. Die erfolgreiche Benutzung der aliphatischen Monocarbonsäure und deren Salze im Zusamemnhang mit CHO-Zellen konnte jedoch auf Grund der vorstehenden Ausführungen nicht vorhergesehen werden, zumal Gorman et al. in Nucleic Acids Research, 11: 7631-7648, 1983, die Benutzung von Natriumbutyrat zur Steigerung der Produktion von CAT nur in serum-haltigem Medium beschreibt.

Es wurde nun gefunden, daß der Zusatz einer C₃-C₄ aliphatischen Carbonsäure oder deren Salze in einer Kultur von transformierten CHO-Zellen in serumfreiem Medium die Proteinausbeute steigert.

Gegenstand der vorliegenden Erfindung ist somit eine Methode zur Steigerung der Produktion von einem Protein aus Kulturen von transformierten CHO-Zellen, welche dadurch gekennzeichnet ist, daß man eine C₃-C₄ aliphatische Monocarbonsäure oder deren Salz der Kultur zusetzt und die Kultivierung in serumfreien Medium durchführt.

Ein weiterer Gegenstand der vorliegenden Erfindung ist eine Methode zur Steigerung der Produktion von Proteinen in Kulturen von transformierten CHO-Zellen, welche dadurch gekennzeichnet ist, daß man die Zellen in Anwesenheit von Thioglykolsäure, Thiodiglykolsäure, L-Cystein, Glutathion, Butyrylcholinbromid, Butyrylcholinchlorid, Nonactinsäure, Furanfettsäure, Ascorbinsäure, Aphidicolin, 6-Hydroxy-4,6-dimethyl-3-hepten-2-on, D-α-hydroxy- substituierte (C₃ oder C₄) aliphatische Mono- oder Dicarbonsäure oder deren Salze kultiviert, die Zellen von der Kultur entfernt und danach in neues serum-freies Kultivierungsmedium gibt und in Anwesenheit von einer C₃-C₄ aliphatischen Monocarbonsäure oder deren Salze kultiviert.

Als Proteine kommen vorzugsweise die Plasminogenaktivatoren, insbesondere rt-PA und deren Mutante, in Betracht. Die Wirkung der Methode ist jedoch unabhängig von den Proteinen und der für diese Proteine codierenden DNA und kann daher, an CHO-Zellen appliziert, die zur Expremierung jeden fremden Proteins benutzt werden.

Mutante rt-PA sind Derivate von t-PA, die eine oder mehrere Aminosäurereste besitzen, die sich von den Aminosäureresten in der entsprechenden Stellung des natürlichen t-PA unterscheiden, oder in dem eine oder mehrere Aminosäurereste des natürlichen t-PA nicht vorkommen. Einige solcher t-PA- Mutanten sind in der Literatur, insbesondere in den oben erwähnten Patentanmeldungen, beschrieben, insbesondere in EP-A-0,199,574 und DE-A-3,708,681.

Als bevorzugte Protein-produktionssteigernde Substanzen kommen Thioglykolsäure, Nonactinsäure, Buttersäure und Propionsäure in Frage.

Nonactinsäure und Verfahren zu deren Herstellung ist in z.B. Helv. Chim. Acta, Vol. XLV, (1962), No. 15-16, S. 129-138; Tetrahedron, Vol. 36, No. 1, S. 46-49 und J. Org. Chem., 1980, 45, S. 4259-4260 beschrieben.

Die Protein-Produktion steigernden Substanzen sind Handelsprodukte oder literaturbekannte Produkte oder können durch bekannte Methoden hergestellt werden.

Butyrylcholinbromid und -chlorid sind Handelsprodukte und z.B. bei der Firma Sigma Chemical Co., St. Louis, Missouri, USA, erhältlich.

Verschiedene Publikationen beschreiben die Furanfettsäuren, z.B. Lipids, 1977, 12(10), 828-36, J. Chem. Soc. Chem. Commun., 1976, (16), S. 630-1, Lipids, 1975, 10(11), S. 695-702, und Fette, Seifen, Anstrichmittel, No. 8, 1986, S. 290-292.

Nutzbare Säuren dieses Types sind auch durch Umsetzung gemäß folgendem Reaktionsschema herstellbar:
a) 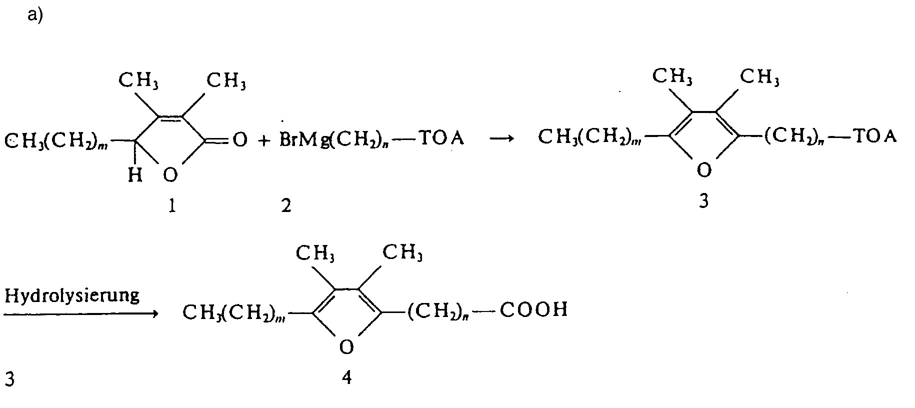 wobei m = 2 bis 5, vorzugsweise 4, n = 7 bis 12, vorzugsweise 8 oder 10, 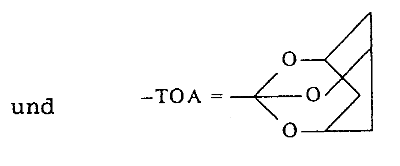
b) 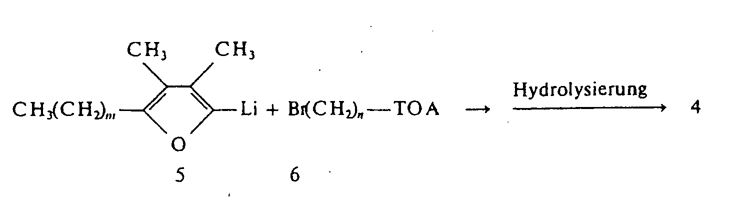 Die Herstellung der Verbindungen 2 und 6 ist von Voß et al., Helv. Chim. Acta: 66, 2294, 1983, beschrieben.
   Vorzugsweise ist m 4 und n 8 oder 10
Die Verbindung 6-Hydroxy-4,6-dimethyl-3-hepten-2-on ist durch Verfahren nach dem Stand der Technik herstellbar. Sie ist auch ein Naturstoff und isolierbar aus Capiscum annuns var angulosum, wie in C.A. 97: 159552f und Eiyo to Shokuryo 1982, 35(2) 95-101, beschrieben. Diese Verbindung ist ebenfalls in der Dissertation "Neue Sekundärstoffe aus Streptomycesten: Isolierung und Strukturaufklärung der Colabomycine, Pyrrolame und des Pyridazomycins" von Grote, R., Universität Göttingen, 1987, beschrieben, sowie ihre Herstellung durch die Züchtung von Streptomyces olivaceus (Stamm Tü 3082, hinterlegt bei der Deutschen Sammlung für Mikroorganismen am 23. November 1987 gemäß Budapester Vertrag unter der Nr. 4309) und Isolierung. Sie hat folgendes NMR-Spektrum:
C₉H₁₆0₂ (156.23)
- EI-MS:: m/e = 138 (M⁺-H₂0, Hochauflösung, C₉H₁₄0, 6%); 123 (M⁺-CH₅0, 8%); 98 (Hochauflösung, C₆H₁₀0, 48%); 83 (100%)
- ¹H-NMR: (200 MHz, CDCl₃): δ = 1.27 (s, 7-H₃ u. 8-H₃); 1.43 (s, breit, HO, tauscht mit MeOD aus); 2.21 (s, 1-H₃); 2.35 (d. J = 1.3 Hz, 9-H₃); 2.32 (s, breit, 5-H₂); 6.13 (s, breit, 3-H) ppm
- ¹³C-NMR: (50.3 MHz, CDCl₃): δ = 22.0 (o, C-9); 30.0 (o, 2C, C-7 u. C-8); 32.0 (o, C-1); 54.4 (u, C-5); 71.1 (u, C-6); 127.2 (o, C-3); 155.1 (u, C-4); 198.6 (u, C-2) ppm
Als Salze der genannten Säuren kommen insbesondere Salze der Alkalimetalle, vorzugsweise der Natrium- und Kaliumsalze, in Frage.

Die vorliegende Erfindung kann in serumhaltigem oder vorzugsweise in serumfreiem Medium durchgeführt werden.

Wenn die Methode in serumhaltigem Medium durchgeführt wird, ist die Verwendung von z.B. Thioglykolsäure, Nonactinsäure oder Furanfettsäure oder deren Salze bevorzugt.

Falls die Methode in serumfreiem Medium durchgeführt wird, wird man vorzugsweise eine C₃-C₄ aliphatische Monocarbonsäure, insbesonere Buttersäure oder Propionsäure, oder deren Salze verwenden.

Es wurde gefunden, daß die Protein-Produktion steigernde Verbindung ihre Wirksamkeit bei Konzentrationen von 10 »M bis 500 mM, z.B. 1 mM bis 10 mM, aufzeigt. Die (C₃-C₄) aliphatische Monocarbonsäure, Thioglykolsäure, Thiodiglykolsäure, L-Cystein und Glutathion oder deren Salze sind insbesondere bei 1 mM bis 10 mM wirksam.

Die Protein-Produktion steigernden Substanzen können am entsprechenden Tage der Kultivierung zugesetzt werden, z.B. am Tag 0, das heißt am Anfang der Kultivierung, bis zum Ende des dritten Tages der Kultivierung.

Man kann auch die Protein-Produktion steigernde Substanz bei mehrfachen Applikationen verwenden. Also kann man in Anwesenheit einer Substanz die Zellen kultivieren, die Zellen von der Kultur entfernen, dann wieder in neues Kultivierungsmedium geben und in Anwesenheit von einer Protein-Produktion steigernden Substanz kultivieren. Bei dieser Mehrfachapplikation zeigen insbesondere C₃-C₄ aliphatischen Monocarbonsäuren oder deren Salze, vorzugsweise Buttersäure und Thioglykolsäure oder deren Salze, einen Steigerungseffekt.

Bei Mehrfachapplikation ist es nicht nötig, daß man die gleiche Substanz für alle Applikationen verwendet, sondern man kann eine der Substanzen in einer Applikation und eine andere in einer weiteren Applikation verwenden. Dieser Mehrfachapplikation zeigt ebenfalls auch einen Steigerungseffekt, wie insbesondere die Verwendung von Aphidicolin in einer Applikation und eine der anderen Substanzen, z.B. Buttersäure, Propionsäure, Butyrylcholinchlorid oder Butyrylcholinbromid oder deren Salze, in einer weiteren Applikation belegen.

Man kann auch Kombinationen von zwei oder mehr Substanzen verwenden. Bevorzugt verwendet man nicht mehr als eine Substanz, die das Zellwachstum hemmt. Man kann z.B. eine C₃-C₄ aliphatische Monocarbonsäure, vorzugsweise Buttersäure und Thioglykolsäure, oder deren Salze verwenden, wobei die Monocarbonsäure oder deren Salz als 2. Substanz der Zellkultur bevorzugt zugegeben wird.

Transformierte chinesische Hamsterovarzellen bedeuten CHO-Zellen, die mit einem für einen erwünschten Proteine codierendem Vektor transformiert sind, wobei sie unter Züchtung das Protein synthetisieren und exprimieren können.

Als transformierte chinesische Hamsterovarzellen kann man z.B. die Zellen, die in den Europäischen Patentanmeldungen 0,093,619 und 0,199,574 und der Deutschen Patentanmeldung 3,708,681 beschrieben sind, verwenden. Durch Kultivierung dieser transformierten CHO-Zellen können rt-PA (EP-A-0,093,619) und gewisse rt-PA-Mutanten (EP-A-0,199,574 und DE-A-3,708,681) hergestellt werden. Die Transformation von CHO-Zellen mit Vektoren, wobei die Zellen andere Proteine exprimieren werden, erfolgt durch literaturbekannte Technik wie z.B. in den oben erwähnten Europäischen und Deutschen Patentanmeldungen.

Als Wirtszelle kann man die unter dem Kennzeichen CHO-Kl-Zelle, die 1957 von Puck aus Biopsie-Material isoliert wurde und 1970 vom American Type Culture Collection (ATCC) unter der Bezeichnung CCL-61 aufgenommen wurde, verwenden. Diese Zellline ist vom ATCC am 23. Dezember 1987 gemäß Budapester Vertrag unter der Nr. CRL 9618 erneut hinterlegt worden.

Man kann die für das erwünschte Protein codierende DNA-Sequenz durch die schon literaturbekannte Technik, wie z.B. in verschiedenen der oben erwähnten Patentanmeldungen, herstellen. Vektoren, die diese Sequenz und auch kontrollierende Sequenzen aufweisen, können nach an und für sich bekannten Methoden konstruiert werden, z.B. unter Verwendung von E.coli K 12294 (ATCC 31446) und E.coli X 1776 (ATCC 31537), die vom ATCC am 23. Dezember 1987 gemäß Budapester Vertrag unter den Nummern 53704 und 53705 erneut hinterlegt worden sind, wie z.B. die Plasmide pETPER und pETPFR, die in EP-A-0,93,619 beschrieben sind. Die CHO-Zellen können durch die durch EP-A-0,093,619, DE-A-3,708,681, EP-A-0,117,059 und EP-A-0,199,574 oder andere literaturbekannte Methoden transformiert werden.

Der Vektor für die rt-PA-Produktion wurde in einem Subklon, CHO-Kl-DUX Bll, integriert (Urlaub et al., Proceedings of the National Academy of Science, USA, 77: 4216-4220, 1980). Diese Zell-Linie, eine Dihydrofolat Reduktase negative Mutante (DHFR-), ist für eine DHFR-abhängige Selektion der transfizierten Zellklone geeignet. Der Vektor für die humane c-DNA kann ein bekannter Plasmid-Vektor pBR-322 oder deren Abkömmling von Escherichia coli sein. Die Promotorregion für das in Frage kommende Gen und für das DHFR-Gen stammt aus SV 40 und die Terminationsregion für beide Gene von einem Hepatitis B-Oberflächenantigen. In den Beispielen wurden CHO-Kl-DUX Bll-Zellen, die durch die Plasmide pETPER oder pEPEFR transformiert sind, verwendet (siehe EP-A-0,117,059).

Als serumfreies Medium stehen verschiedene bekannte Produkte zur Verfügung (siehe z.B. BMFT-Statusseminar 12, 13. November 1985, Bundesministerium für Forschung und Technologie, 5300 Bonn 2, Seite 111-120). Im folgenden Beispiel wird ein DMEM/Ham's F 12 Medium (1:1) (Gibco) als serumfreies Medium verwendet. Als Zusatz wird fötales Kälberserum (FKS) in einer Konzentration von 7,5% oder 1-2% verwendet.

Die Zellkulturen werden bei 37°C in einem Hereaus-Brutschrank inkubiert und mit einem Kohlendioxid-Luftgemisch von 7,5% CO₂ in einer relativen Luftfeuchtigkeit von 100% belüftet. Je nach Verfahrensansatz werden unterschiedliche Kulturgefäße benutzt: Spinnergefäße für 100-1000 ml Medium, Roux-Schalen (Nunc) 175 cm² (70-100 ml Medium), 75 cm² (30-50 ml Medium), 25 cm² (7-10 ml Medium) und Mehrfachkulturschalen von Costar mit 2 cm² für 1 ml Kulturen. Für die Stammhaltung werden die Zellen in einer Dichte von 0,2-0,3 x 10⁶ Zellen/ml eingesetzt und alle 2-3 Tage subkloniert.

Die Protein-Produktion stimulierenden Substanzen können zu den verschiedenen Zeitpunkten jeweils in einer 1:100 Verdünnung zugegeben werden, d.h. sie wurden zuerst in Medium gelöst oder, wenn sie schwerlöslich sind, erst in Äthylalkohol gelöst und danach in Medium zugesetzt und dann das diese Substanz enthaltende Medium dem zellenthaltenden Medium im Verhältnis 1:100 v/v zugegeben.

In den folgenden Beispielen wurden, um die Stimulierung der rt-PA-Synthese hervorzurufen, Zellen einer exponentiell wachsenden Kultur entnommen und in Plastikröhrchen (Falcon) 10 Min. bei 1000 x g in einer Beckmann T 3-6 Zentrifuge abzentrifugiert. Nach Dekantieren des Überstandes wurde das Zellpellet mit frischem Medium resuspendiert.

Für die Produktion von rt-PA in serumhaltigem Medium wurde frisches F12/DMEM-Medium + 7,5% FKS + Gentamycin verwendet. Die resultierende Suspension kann in 2 cm² 24 Wells-Platten bei einer Zelldichte von 0,2-0,3 x 10⁶ Zellen/ml angesetzt werden.

Für die Produktion von rt-PA in serumfreiem Medium wurden die Zellen zuvor 3-4 Tage in einem Medium von 1-2% FKS adaptiert, wieder abzentrifugiert und das Zellpellet in serumfreiem Medium resuspendiert und anschließend in 2 cm² 24 Wells-Platten in einer Zelldichte von 0,4-0,5 x 10⁶ Zellen/ml überführt.

### Quantitative t-PA-Bestimmungen

Aliquots von Zellüberständen und Zellextrakten wurden zu verschiedenen Zeitpunkten abgenommen bzw. hergestellt. Die Messung der Proben erfolgte entweder sofort oder nach Aufbewahrung bei -20°C mit folgenden Methoden:

### BESTIMMUNGSMETHODE

### 1 - Direkter Chromogener Assay (DCA)

Von dem synthetischen Peptidsubstrat S-2288 (D-H-Ile-Pro-Arg-p-Nitroanilin) der Firma Kabi Diagnostica wird durch das rt-PA p-Nitroanilin als Reaktionsprodukt gebildet. Die Aktivität von rt-PA ist zur Bildung von p-Nitroanilin proportional, die bei 405 mn, 37°C, gemessen wird.

Die spektrophotometrische Bestimmung der Enzymkinetik wurde mit einem ACP-5O10-Analysengerät der Firma Eppendorf automatisch durchgeführt.

Die Aktivität des Enzyms wurde anhand dieses vorgegebenen Standards berechnet. Eine rt-PA-Standardreihe von 1-15 »g/ml wurde mit einem Laborstandard hergestellt, der zu 72% aus 1-kettigem Material besteht.

Die Substratlösung (100 mM Tris/106 mM NaCl) wurde in einer Konzentration von 50 mM eingesetzt.

### 2 - ELISA

Mit Hilfe eines ELISA (Enzyme-linked-immuno-sorbent-assay) wurden die rt-PA-Proben des Überstandes und des Zellextraktes quantitativ bestimmt. Als Standard diente ein Laborstandard in einer Konzentrationsreihe von 0,038-5 »g/ml. Die Proben wurden 1:10 bis 1:1000 vorverdünnt.

### Charakterisierung der transformierten CHO-Zellen

### Zellwachstum

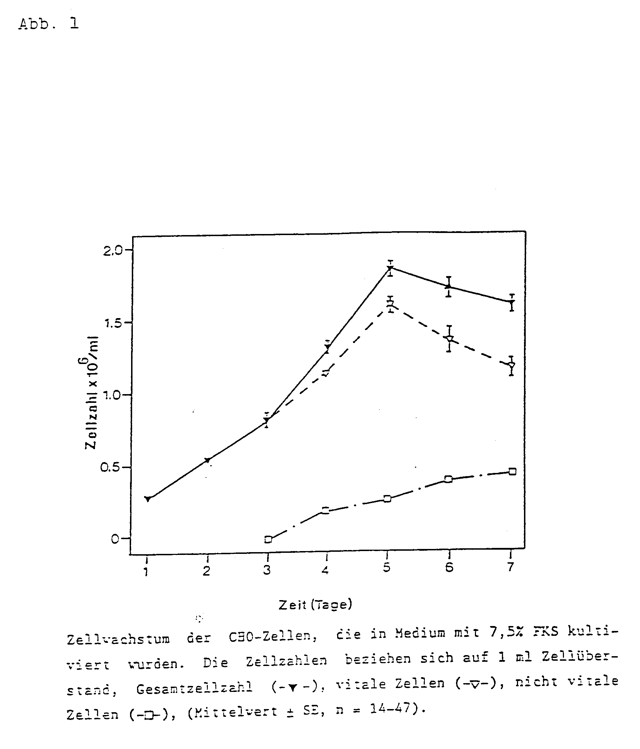

Die CHO-Zellen werden mit 0,2-0,3 x 10⁶ Zellen/ml in Kulturschalen (in Medium mit 7.5% FKS) angeimpft und vermehren sich zu Anfang sehr langsam. Erst nach einer lag-Phase von 24 h beginnt das logarithmische Zellwachstum. Bei einer Zelldichte von 1,3 ± 0,04 x 10⁶ Zellen/ml treten am 4. Tag die ersten nicht vitalen Zellen in der Kultur auf; ihr Anteil an der Gesamtzellzahl beträgt 13,2 ± 1,3%. Die maximale Zelldichte wird am 5. Tag mit 1,8 ± 0,05 x 10⁶ Zellen/ml erreicht (Abb. 1).

Die Zahl der lebenden Zellen stellt nach 7 Tagen noch 72 ± 3% der gesamten Zellmasse dar.

Die stationäre Phase, die sich an das exponentielle Zellwachstum anschließt, wird von verschiedenen Faktoren eingeleitet. Durch eine hohe Populationsdichte und Verbrauch essentieller Nährstoffe teilen sich die Zellen nicht weiter. Die Dauer der stationären Phase variiert stark und ist abhängig von Medienkomponenten wie Serum. Eine Anhäufung von Säuren und toxischen Stoffwechselprodukten sowie Veränderungen von Milieufaktoren (saurer pH, niedriger 0₂-Partialdruck, mangelnde Durchlüftung), bedingen ebenfalls das Absterben der Zellen.

### rt-PA-Produktion in CHO-Zellen

Zwischen Zellwachstum und rt-PA-Synthese besteht eine lineare Beziehung.

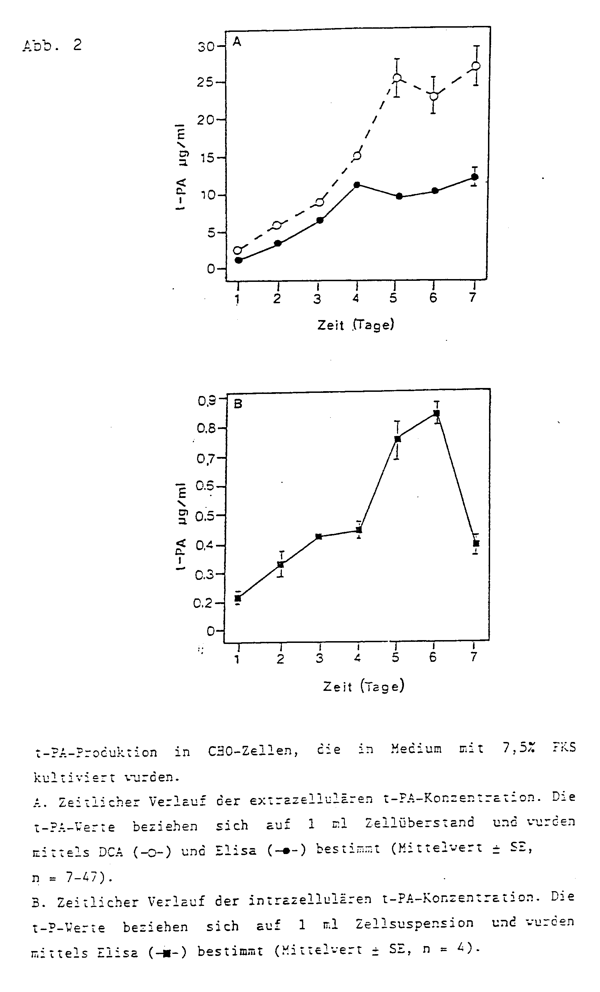

Der Zeitverlauf der rt-PA-Produktion zeigt 2 Phasen. In der 1. Phase steigt die rt-PA-Konzentration im Überstand stetig an. In der 2. Phase kommt die Enzymsynthese praktisch zum Stillstand und die rt-PA-Konzentration in Medium bleibt annähernd konstant (Abb. 2A). Die maximale rt-PA-Konzentration am 4. Tag beträgt 11,1 ± 0,7 »g/ml im Elisa. Die gemessenen rt-PA-Konzentrationen liegen mit dem chromogenen assay etwas höher, was vermutlich durch 2-kettiges rt-PA verursacht wird, mit der Elisa-Methode jedoch verlaufen die Kurven mit beiden Nachweismethoden bis zum 4. Tag parallel.

Die intrazelluläre rt-PA-Konzentration verläuft ebenfalls parallel zum Zellwachstum und ist relativ gering bezogen auf die gesamte synthetisierte rt-PA-Menge. Am 4. Tag beträgt deren Anteil 7% an der Gesamtaktivität (Abb. 2B).

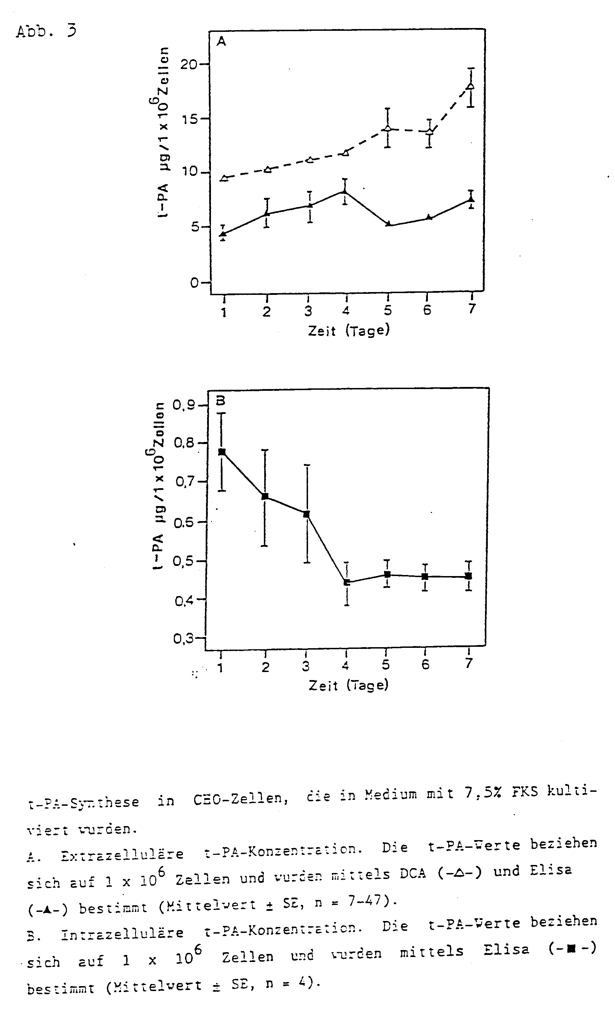

Wie Abb. 3A zeigt, steigt die rt-PA-Synthese, bezogen auf die Zellzahl, bis zum 4. Tag leicht an; mit der Elisa-Methode wird ein Maximum von 8,1 ± 1,2 »g rt-PA/1 x 10⁶ Zellen bestimmt. Zellulär gebundenes rt-PA wurde nur mit der Elisa-Methode bestimmt, da diese geringen rt-PA-Konzentrationen (< 1 »g) mittels DCA nicht zu bestimmen sind.

Der intrazelluläre rt-PA-Gehalt, bezogen auf die Zellzahl, ist während der log-Phase des Zellwachstums etwas höher und liegt zwischen 500 und 900 ng rt-PA / 1 x 10⁶ Zellen, ab dem 4. Tag liegt der rt-PA-Wert bei ca. 450 ng / 1 x 10⁶ Zellen (Abb. 3B).

### Einfluß von Serum und Serumfaktoren im Medium auf das Zellwachstum und die rt-PA-Produktion in CHO-Zellen

Die Zellen wurden mit 0,25 x 10⁶ Zellen/ml und verschiedenen Serumkonzentrationen in Medium angesetzt.

Eine Serumkonzentration unter 5% im Medium wirkt sich auf die Zellteilung gleichfalls negativ aus, die Produktivität wird hingegen nur gering beeinflußt.

Der Einfluß der Serumkonzentration in Medium auf das Zellwachstum und die rt-PA-Produktion nach 48 h-Inkubation der Zellen ist in Tabelle 1 dargestellt. Die Werte sind in % der maximalen Werte (7,5% FKS in Medium) angegeben. Der Maximalwert für die Zellzahl liegt bei 0,56 ± 0,03 x 10⁶ Zellen und für die rt-PA-Konzentration 5,7 ± 0,5 »g/ml. Die rt-PA-Bestimmungen wurden mittels DCA durchgeführt (Mittelwert ± SE, n = 4).

**TABELLE 1**

| % FKS | Zellzahl | rt-PA |
|---|---|---|
| 40 | 61,5 ± 2,8 | 59,2 ± 3,9 |
| 20 | 79,4 ± 3,8 | 81,2 ± 2,4 |
| 10 | 88,6 ± 5,3 | 90,3 ± 6,7 |
| 7,5 | 100,0 ± 6,4 | 100,0 ± 7,9 |
| 5 | 72,4 ± 4,9 | 87,0 ± 11,3 |
| 1 | 43,1 ± 1,5 | 79,2 ± 6,7 |
| 0 | 37,5 ± 1,8 | 75,4 ± 4,6 |

### Zellkultur in serumfreiem Medium

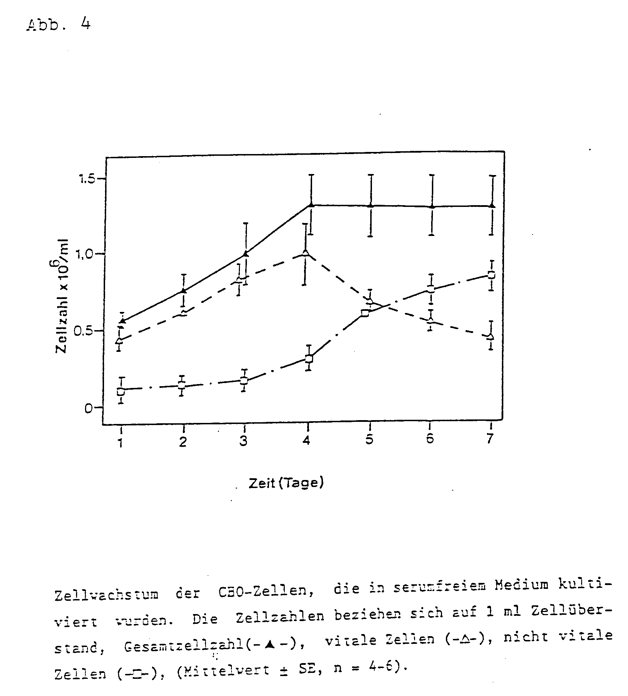

Die Zellen wurden in serumfreiem Medium in einer Zelldichte von 0,4-0,5 x 10⁶ Zellen/ml angesetzt. Die Zellen teilen sich bis zum 4. Tag, und die höchste Lebendzellzahl wird ebenfalls am 4. Tag mit 1,0 ± 0,2 x 10⁶ Zellen/ml erreicht (Abb. 4).

### rt-PA-Produktion in serumfreiem Medium

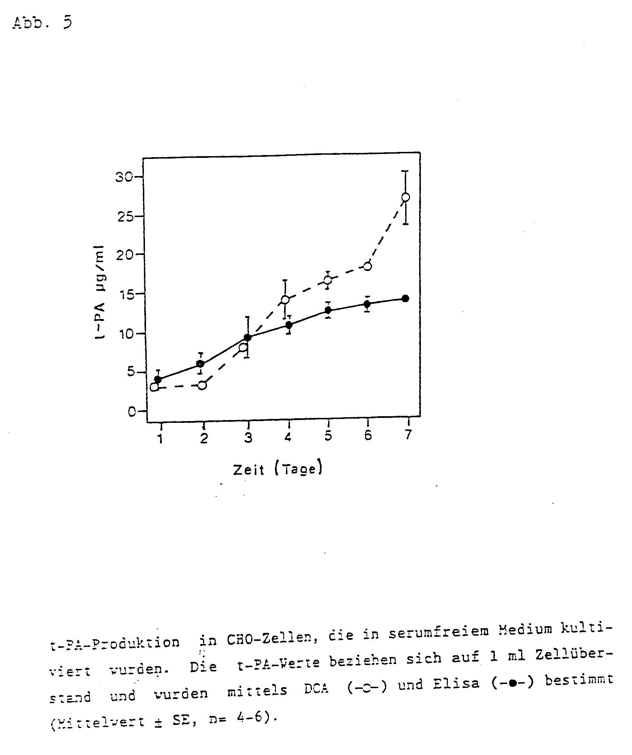

Die rt-PA-Konzentration im Überstand steigt bis zum 7. Tag ständig an und erreicht mit der Elisa-Methode ein Maximum von 13,6 ± 0,54 »g/ml. Die, mittels DCA ermittelten t-PA-Werte, liegen am 1.-3. Tag unter, und ab dem 4. Tag über den rt-PA-Werten, die mittels Elisa bestimmt wurden (Abb. 5).

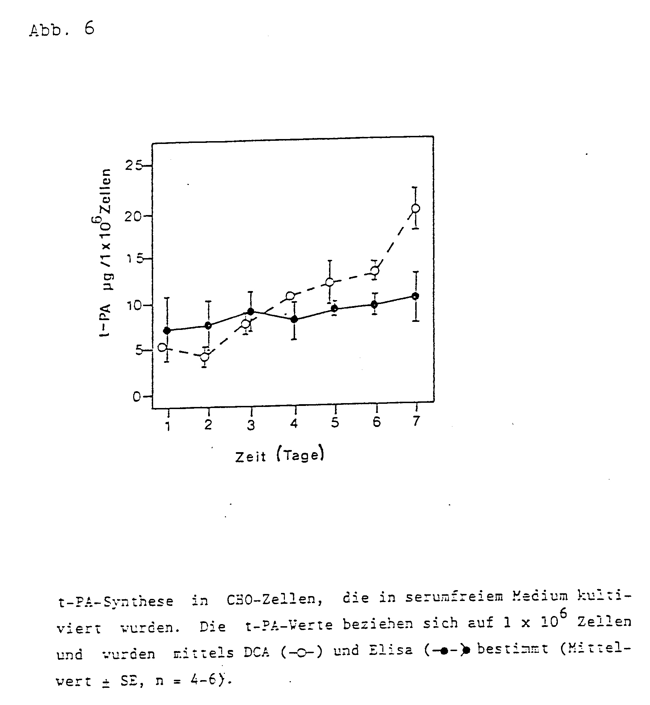

Die rt-PA Synthese pro Zelle ist in serumfreiem Medium ungefähr gleich hoch wie in Medium mit Serum und liegt zwischen 7,2 ± 3,6 und 10,3 ± 2,8 »g/ 1 x 10⁶ Zellen (Elisa) (Abb. 6).

Der prozentuale Anteil von rt-PA in serumfreiem Medium an der Gesamtproteinmenge des Überstandes liegt zwischen 10,9 ± 1,0 und 12,5 ± 1,4%. Die Ergebnisse sind in Tabelle 2 angegeben. Die t-PA-Werte wurden mittels Elisa bestimmt (Mittelwert ± SE, n = 4).

**TABELLE 2**

| Tag | Protein »g/1x10⁶ Zellen | rt-PA »g/1x10⁶Zellen |
|---|---|---|
| 1 | 71,0 ± 8,0 | 7,2 ± 3,6 |
| 3 | 81,8 ± 9,4 | 9,0 ± 2,3 |
| 5 | 82,0 ± 12,2 | 9,3 ± 0,8 |

### Effekt von aliphatischen Monocarbonsäuren

Steigerung der rt-PA-produktion in CHO-Zellen in Medium mit 7,5% FKS durch verschiedenen aliphatischen Monocarbonsäuren sind in Tabelle 3 angegeben. Alle Säuren wurden in einem Konzentrationsbereich von 10 mM bis 500 mM verwendet. Der optimale Dosisbereich für alle Monocarbonsäuren liegt zwischen 1 bis 10 mM. Die Wirksamkeit der Säuren hängt von der Kettenlänge ab.

Alle wirksamen Monocarbonsäuren hemmen das Zellwachstum. Propion-, Butter- und Isobuttersäure sind in einem größeren Konzentrationsbereich wirksam, da die konzentrationsabhängige Inhibition des Primärstoffwechsels, die zur Hemmung des Zellwachstums führt, sich mit der Steigerung der t-PA-Synthese überlagert.

Die Werte in Tabelle 3 beziehen sich auf 1 ml Zellüberstand und sind in % gegenüber der Kontrolle (0%) angegeben. Die t-PA-Bestimmung erfolgte mittels DCA (Mittelwert ± SE, n = 3-24). Der maximale Wert bezieht sich auf eine Einzelprobe. Daten für die einzelnen Proben sind in Klammern angegeben.

**TABELLE 3**

| Monocarbonsäure | optimaler Dosisbereich | % Steigerung der rt-PA-Synthese | |
|---|---|---|---|
| | | Maximum | Durchschnitt |
| Propionsäure | 5-10 mM (10 mM) | 45,5 | 28,0 ± 2,5 |
| Buttersäure | 1-5 mM ( 1 mM) | 68,3 | 41,9 ± 3,8 |
| Isobuttersäure | 0,6-10 mM (10 mM) | 32,9 | 16.6 ± 2,8 |

Die Durchschnittswerte beziehen sich auf mehrere Produktionsgänge von 3 bis 4 Tagen, wobei die Monocarbonsäuren am Tag 0 bis Tag 3 der Kultivierung zugegeben worden sind, wie unten in Tabelle 3A dargestellt. Daten für die einzelnen Proben sind in Klammern angegeben.

**TABELLE 3A**

| Monocarbonsäure | Zugabetage | Probetage | n |
|---|---|---|---|
| Propionsäure | 0-3 (2) | 1-4 (3) | 24 |
| Buttersäure | 1 (3) | 2-4 (3) | 18 |
| Isobuttersäure | 0-2 (3) | 2-4 (3) | 11 |
| n = Anzahl der Produktionsgänge | | | |

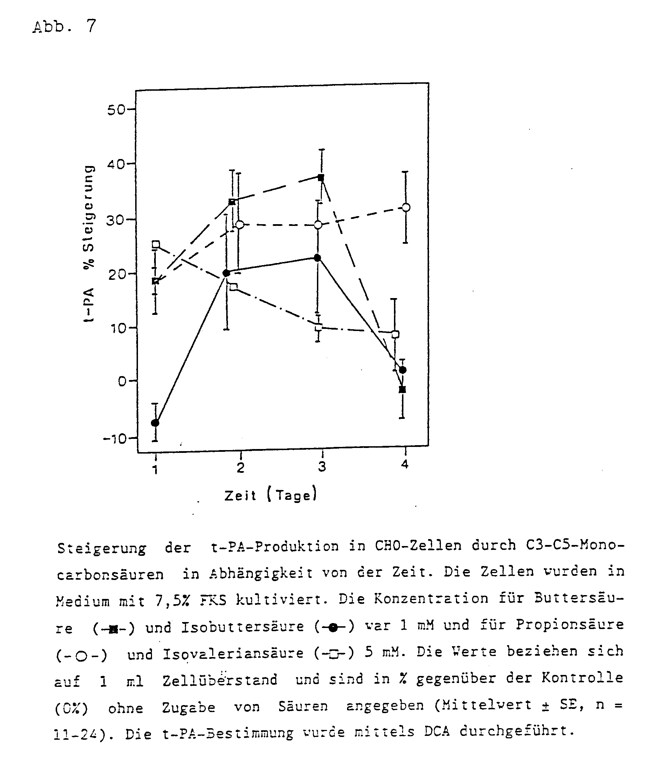

In Abb. 7 ist der zeitliche Verlauf der rt-PA-Produktionssteigerung in Medium mit 7,5% FKS durch C₃-C₅-Monocarbonsäuren dargestellt. Nach 24 h ist schon eine Stimulierung der rt-PA-Produktion von 18,1 ± 5,7% (C₄) bis 24,8 ± 0,5% (C₅) nachweisbar. Bei Isobuttersäure zeigte sich eine Produktionssteigerung erst nach 48 h. Eine maximale Steigerung der rt-PA-Produktion wurde für die C₃- und die C₄-Carbonsäure am 3. Tag erreicht, mit 37,2 ± 5,1% für Na-Butyrat. Die Produktionssteigerung ist für die Butter- und Isobuttersäure nur kurzzeitig, ein Effekt ist am 4. Tag nicht mehr zu beobachten. Die steigernde Wirkung von Propionsäure und Isovaleriansäure hingegen ist am 4. Tag noch vorhanden und manifestiert sich in einer Steigerung der t-PA-Produktion um 31,8% für C₃ und 8,5% für C₅.

Die Effekte sind außerdem vom physiologischen Zustand der Zelle abhängig. Während des exponentiellen Wachstums sind die Zellen für eine Stimulierung am empfindlichsten.

In Tabelle 4 ist die Steigerung der rt-PA-Produktion in CHO-Zellen in Medium mit 7,5% FKS durch C₃-C₄-Monocarbonsäuren in Abhängigkeit vom Applikationstag der Carbonsäuren nach 48 h Wachstum der Zellen in Medium mit 7,5% FKS dargestellt. Die Konzentration war für Buttersäure 1 mM und für Propionsäure 5 mM. Die Werte beziehen sich auf 1 ml Zellüberstand und sind in % gegenüber der Kontrolle (0%) angegeben (Mittelwert ± SE, 3 = 3-5). Die t-PA-Bestimmung wurde mittels DCA durchgeführt.

**TABELLE 4**

| Applikationstag | C₃ | C₄ | C₄-I |
|---|---|---|---|
| 0 | 28,7 ± 9,3 | 33,0 ± 5,4 | 20,1 ± 10,7 |
| 1 | 24,4 ± 4,1 | 40,2 ± 12,2 | 25,3 ± 5,1 |

### Effekte der Buttersäure auf die rt-PA-Produktion in CHO-Zellen

Der Stimulierungseffekt ist an einer Steigerung sowohl der intrazellulären rt-PA-Konzentration als auch der extrazellulären rt-PA-Konzentration zu verfolgen.

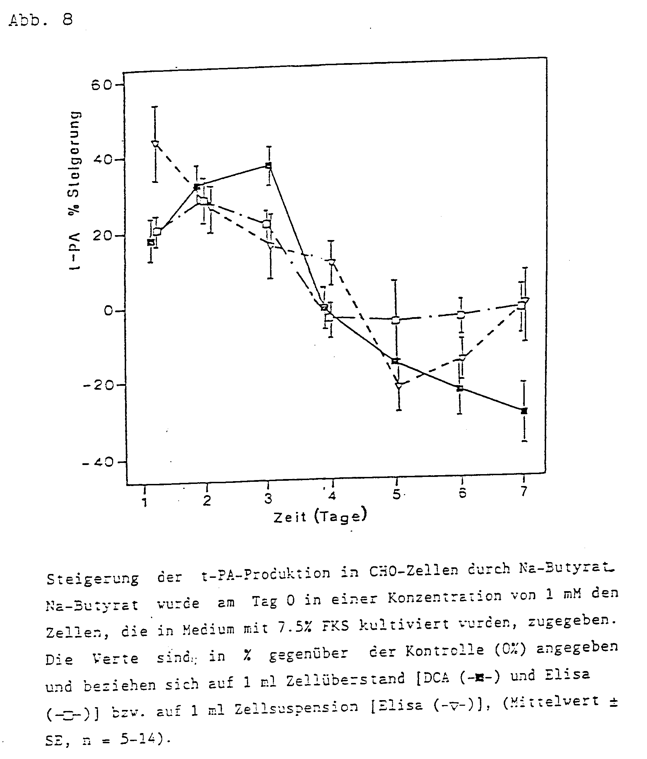

Wie aus Abb. 8 hervorgeht, ist die Zunahme des intrazellulären rt-PA-Gehaltes in Butyrat-behandelten Kulturen nach 24 h-Inkubation gegenüber der Kontrolle am größten (43,8 ± 9,8%). Im Überstand fällt die prozentuale Steigerung nach 24 h etwas geringer aus mit durchschnittlich 19,2% (DCA und Elisa) gegenüber den Kontrollwerten. Dieser Effekt hält im Überstand 72 h an.

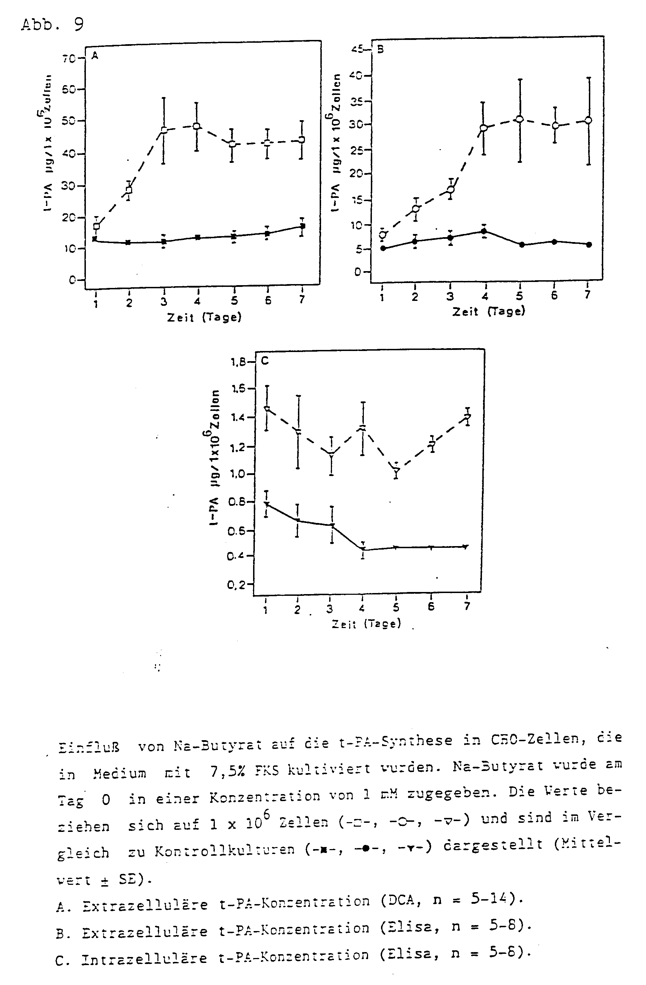

In Abb. 9A-C ist die rt-PA-Synthese, bezogen auf die Zellzahl dargestellt, und sie zeigen, daß die Stimulierung der rt-PA-Synthese durch Na-Butyrat trotz der Hemmung des Zellwachstums über 7 Tage anhält. Während der ersten 4 Tage nimmt die Enzymsynthese pro Zelle stetig zu. Die rt-PA-Konzentration steigt von 7,5 ± 1,1 »g bis 28,9 ± 5,3 »g rt-PA/ 1 x 10⁶ Zellen (Elisa) im Überstand an (Abb. 8A). Mit der DCA-Methode wird ein maximaler rt-PA-Gehalt von 47,4 ± 7,4 »g t-PA/1 x 10⁶ Zellen bestimmt (Abb. 9B). Gleichzeitig nimmt der intrazelluläre rt-PA-Gehalt ab und fällt von 1,5 ± 0,2 »g t-PA/ 1 x 10⁶ Zellen am 5. Tag ab (Abb. 9C).

In Tabelle 5 ist die Stimulierung der rt-PA-Synthese durch Na-Butyrat in Medium mit 7,5% FKS bezogen auf die Zellzahl angegeben. Na-Butyrat wurde am Tag 0 in einer Konzentration von 1 mM zugegeben. Die Werte an den Tagen 1-4 sind in % gegenüber der Kontrolle (0%) angegeben und beziehen sich auf 1 x 10⁶ Zellen. Der rt-PA-Gehalt im Überstand und im Zellextrakt wurde mittels Elisa bestimmt (Mittelwert ± SE, n = 5-10).

**TABELLE 5**

| Tag | intrazellulär | extrazellulär |
|---|---|---|
| 1 | 90,9 ± 9,5 | 68,5 ± 12,4 |
| 2 | 99,7 ± 26,3 | 18,8 ± 16,1 |
| 3 | 93,9 ± 31,4 | 223,4 ± 77,4 |
| 4 | 209,8 ± 41,4 | 280,7 ± 85,9 |

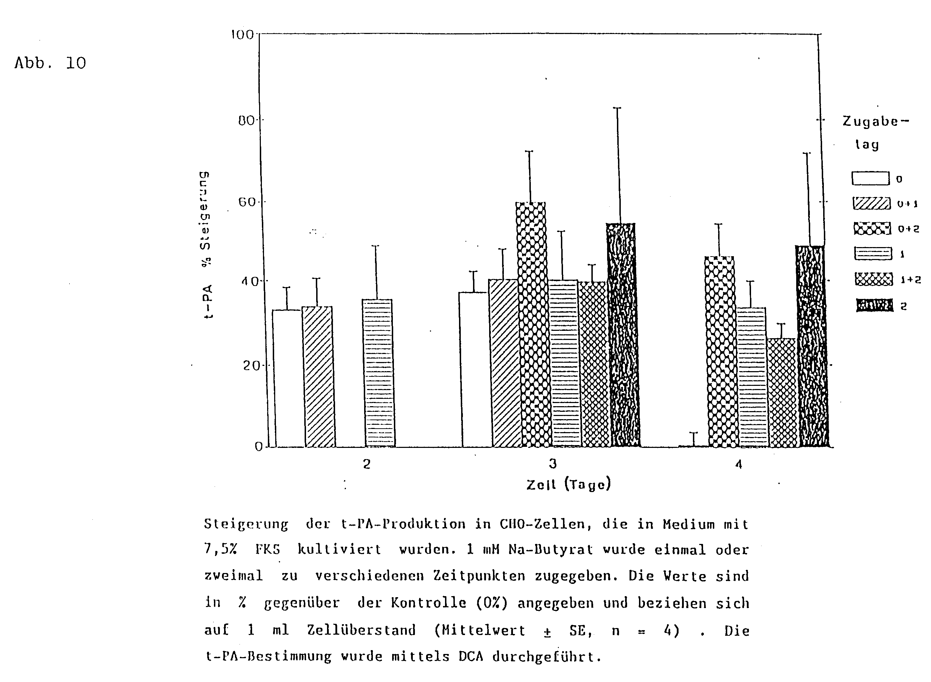

Eine optimale Produktionssteigerung wurde erreicht, wenn Na-Butyrat während des exponentiellen Wachstums der Zelle zugegeben wurde. Der maximale Effekt von Na-Butyrat tritt bei einem Applikationszeitraum von Tag 0 bis 2 immer am 3. Tag auf (Abb. 10).

Durch eine Applikation von Na-Butyrat alle 2-3 Tage nach Mediumwechsel in derselben Zellpopulation konnte die rt-PA-Produktion über längere Zeit auf einem höheren Niveau gehalten werden.

In Tabelle 6 ist die Steigerung der rt-PA-Produktion in CHO-Zellen, die in Medium mit 7,5% FKS kultiviert wurden, dargestellt. 1 mM Na-Butyrat wurde bei jedem Mediumwechsel bzw. nur am Tag 0 zugegeben. Die Werte von 2, 4, 7 bzw. 9 Tagen nach Mediumwechsel sind in % gegenüber der Kontrolle (%) angegeben und beziehen sich auf 1 ml Zellüberstand. Die t-PA-Bestimmung wurde mittels DCA durchgeführt (Mittelwert ± SE, n = 3).

**TABELLE 6**

| Mediumwechsel Tag | Applikation Tag 0, 2, 4, 7 |
|---|---|
| 2 | 33,0 ± 5,4 |
| 4 | 107,4 ± 7,6 |
| 7 | 96,6 ± 5,8 |
| 9 | 62,0 ± 4,8 |

### Effekte der Buttersäure auf die rt-PA-Produktion in CHO-Zelle in serumfreiem Medium

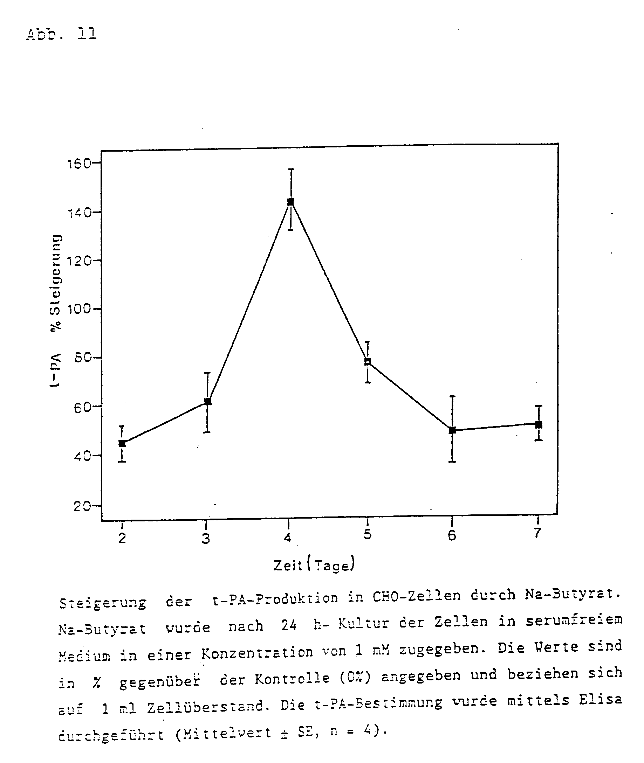

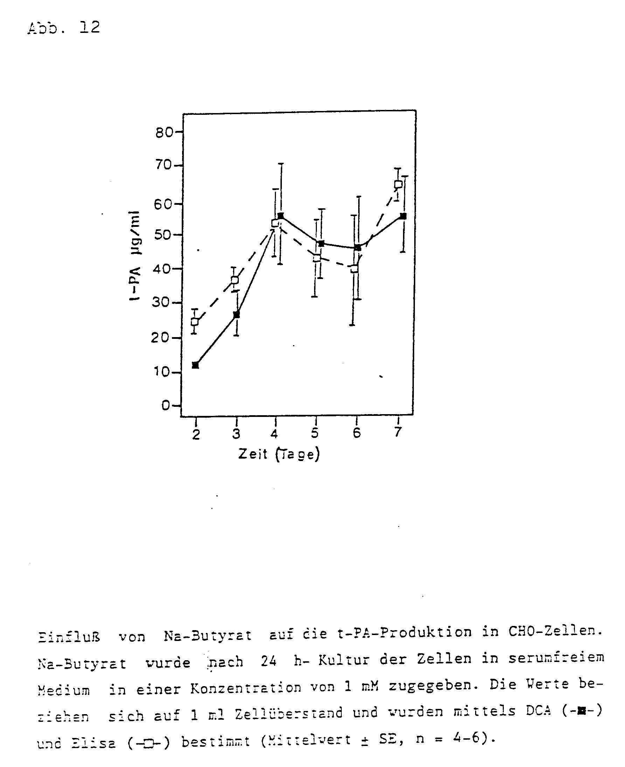

Im Gegensatz zu dem Kurzzeiteffekt der Butyrat-stimulierten rt-PA-Synthese in serumhaltigem Medium hält der Effekt in serumfreiem Medium länger an und beträgt 1 Tag nach Applikation von Na-Butyrat 44,1 ± 7,1% gegenüber der Kontrolle. Eine maximale Steigerung wurde 3 Tage nach Applikation von Na-Butyrat erreicht mit 143,3 ± 12,8%. Der Steigerungseffekt ist noch 6 Tage nach Na-Butyrat-Zugabe mit 49,9 ± 7,0% nachzuweisen (Abb. 11 und 12).

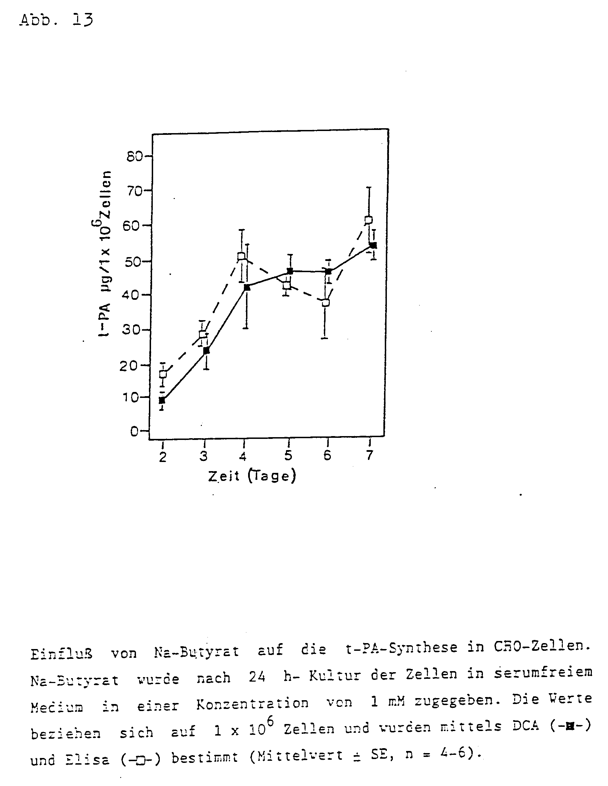

Die rt-PA-Synthese, bezogen auf 1 x 10⁶ Zellen, ist in serumfreiem Medium ebenfalls höher als in serumhaltigem Medium und steigt auf über 50 »g/ 1 x 10⁶ Zellen (Abb. 13).

### Effekte der Propionsäure

Die C₃-Carbonsäure zeigt sehr ähnliche Effekte auf die rt-PA-Synthese wie die Buttersäure. Die prozentuale Steigerung fällt jedoch ca. 14% niedriger aus und für die rt-PA-Stimulierung sind höhere Konzentrationen (5-10 mM) notwendig. Weiterhin hemmt Propionsäure in geringerem Maße das Zellwachstum und führt daher zu einer längeranhaltenden Steigerung der rt-PA-Synthese.

Eine Stimulierung der rt-PA-Synthese durch Propionsäure scheint, im Gegensatz zur Buttersäure, sehr spezifisch zu sein, da in serumfreiem Medium der extrazelluläre Proteingehalt gegenüber der Kontrolle nur gering erhöht wurde und der Einbau von [³H]-Leucin nur geringfügig gesteigert wurde.

### Effekte der Ascorbinsäure

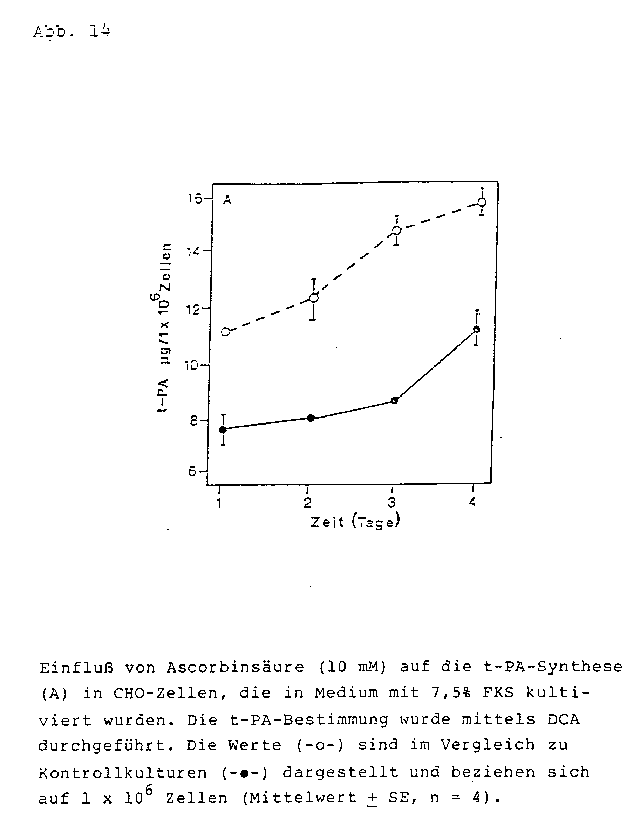

Die Wirkung der Ascorbinsäuren bei 10 mM auf die rt-PA-Produktion hält 72 h an und zeigt bei einer Applikation am Tag 0 eine 15,2 ± 3,1%-Steigerung der rt-PA-Synthese nach 24 h (Abb. 14).

### Effekte langkettiger Fettsäuren auf die rt-PA-Produktion in CHO-Zellen

Es wurde gefunden, daß auch langkettige Fettsäuren wie Nonactinsäure mit 10C-Atomen und Furanfettsäuren eine Steigerung hervorrufen konnten (Tabelle 7). In diesem Beispiel wurde eine Furanfettsäure gemäß Formula 4, in der m = 4 und n = 8, verwendet.

In serumfreiem Medium war jedoch keine Steigerung der rt-PA-Synthese bzw. der Proteinsynthese nachzuweisen.

Die Steigerung der rt-PA-Produktion in CHO-Zellen durch langkettige Fettsäuren ist in Tabelle 8 dargestellt. Die Zellen wurden in Medium mit 7,5% FKS kultiviert. Die Säuren wurden in einem Konzentrationsbereich von 10 »M bis 10 mM getestet, wobei eine Konzentration von 1 mM optimal war. Die Werte beziehen sich auf 1 ml Zellüberstand und sind in % gegenüber der Kontrolle (0%) angegeben (Mittelwert ± SE, n = 4-9). Der maximale Wert bezieht sich auf eine Einzelprobe. Die rt-PA-Bestimmung wurde mittels DCA durchgeführt.

**TABELLE 7**

| Fettsäure | % Steigerung der rt-PA-Synthese | |
|---|---|---|
| | Maximum | Durchschnitt |
| Nonactinsäure | 58,3 | 30,3 ± 6,5 |
| Furanfettsäure | 32,1 | 19,0 ± 3,2 |

Die Durchschnittswerte beziehen sich auf mehrere Produktionsgänge von 4 Tagen, wobei die Säuren am Tag 0 zugegeben wurden, wie in Tabelle 8A dargestellt. Daten für die Einzelproben sind in Klammern angegeben.

**TABELLE 7A**

| Fettsäuren | Zugabetag | Probetag | n |
|---|---|---|---|
| Nonactinsäure | 0 (0) | 1-4 (2) | 4 |
| Furanfettsäure | 0 (0) | 1-4 (2) | 9 |

### Effekte von Thiolen und Sulfiden auf die rt-PA Produktion in CHO-Zellen

Vier schwefelhaltige Verbindungen, die Derivate oder Substitutionsprodukte von Carbonsäuren darstellen, zeigten eine Steigerung der rt-PA-Produktion zwischen 16% und 31,2%, ohne das Zellwachstum negativ zu beeinflussen (Tabelle 8). Die substituierten Carbonsäuren zeigten ihre optimale Wirkung im gleichen Konzentrationsbereich (1-10 mM) wie die anderen wirksamen Carbonsäuren. Nur Glutathion war in einem niedrigerem Konzentrationsbereich von 1-10 »M effektiv. Thioglykolsäure erwies sich als bevorzugter Stimulator der rt-PA-Synthese in CHO-Zellen.

In diesem Beispiel wurden die Zellen in Medium mit 7,5%FKS kultiviert. Alle Substanzen wurden in einem Konzentrationsbereich von 1 »M bis 10 mM getestet. Die Werte in Tabelle 9 beziehen sich auf 1 ml Zellüberstand und sind in % gegenüber der Kontrolle (%) angegeben (Mittelwert ± SE, n = 6-19). Der maximale Wert bezieht sich auf eine Einzelprobe. Die rt-PA-Bestimmung wurde mittels DCA durchgeführt. Die Konzentration der Substanz ist in Klammern angegeben.

**TABELLE 8**

| Thiole und Sulfide | optimaler Dosisbereich | % Steigerung der rt-PA-Synthese | |
|---|---|---|---|
| | | Maximum | Durchschnitt |
| Thioglykolsäure | 1 mM | 66,4 | 31,2 ± 3,2 |
| Thiodiglykolsäure | 1 mM | 31,0 | 18,0 ± 3,8 |
| L-Cystein | 1-10 mM ( 1mM) | 41,4 | 20,9 ± 3,4 |
| Glutathion | 1-10 »M (10»M) | 30,6 | 16,0 ± 2,0 |

Die Durchschnittswerte beziehen sich auf mehrere Produktionsgänge von 3 Tagen, wobei die Substanzen vom Tag 0 bis Tag 2 zugegeben worden sind wie in Tabelle 9A angegeben. Daten für die einzelnen Proben sind in Klammern dargestellt.

**TABELLE 8A**

| Fettsäuren | Zugabetag | Probetag | n |
|---|---|---|---|
| Thioglykolsäure | 0-2 (1) | 1-3 (2) | 19 |
| Thiodiglykolsäure | 1 (1) | 2-3 (3) | 9 |
| L-Cystein | 0-1 (0) | 1-3 (2) | 11 |
| Glutathion | 0 (0) | 2-3 (2) | 6 |

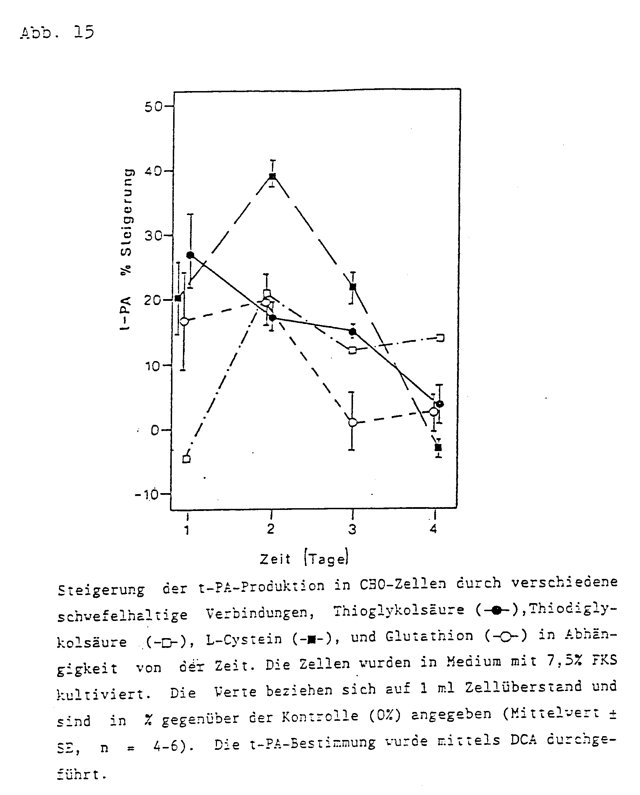

Die Vorteile von Thiocarbonsäuren gegenüber Monocarbonsäuren ist das Fehlen eines inhibitorischen Effekts auf die Zellproliferation, jedoch konnte die Produktion von rt-PA mit diesen Substanzen nicht weiter gesteigert werden als mit Buttersäure. Die Dauer des Steigerungseffektes ist ebenso kurz und erreicht den Maximalwert nach 24 h für Thioglykolsäure und nach 48 h für Cystein. Nach 4 Tagen Inkubation der Zellen ist der Effekt auf die rt-PA-Produktion nur noch gering (Abb. 15).

In serumfreiem Medium ist die stimulierende Wirkung auf die rt-PA-Synthese aber geringer als in Medium mit Serum, da alle Substanzen die Zellteilung zwischen 10% und 20% hemmen.

### Effekte der Thioglykolsäure

Die rt-PA-Produktion ist nach der Thioglykolsäure-Behandlung 72 h erhöht und vom Applikationstag unabhängig. Die prozentuale Steigerung der Enzymsynthese ist jeweils nach 24 h am höchsten und fällt dann kontinuierlich ab.

In Tabelle 9 ist die Steigerung der rt-PA-Produktion in CHO-Zellen, die 24-96 h in Medium mit 7,5% FKS und 1 mM Thioglykolsäure kultiviert wurden, in Abhängigkeit vom Applikationstag, dargestellt. Die Werte beziehen sich auf 1 ml Zellüberstand und sind in % gegenüber der Kontrolle (0%) angegeben (Mittelwert ± SE, n = 4-6). Die rt-PA-Bestimmung wurde mittels DCA durchgeführt.

**TABELLE 9**

| Zeit (h) | Applikationstag | | |
|---|---|---|---|
| | 0 | 1 | 2 |
| 24 | 27,6 ± 5,6 | 34,7 ± 10,9 | 34,5 ± 7,5 |
| 48 | 16,9 ± 2,3 | 17,1 ± 3,6 | 16,3 ± 8,6 |
| 72 | 14,7 ± 1,0 | 10,2 ± 2,7 | 9,4 ± 1,7 |
| 96 | 3,4 ± 3,0 | 2,7 ± 0,8 | 0,5 ± 1,0 |

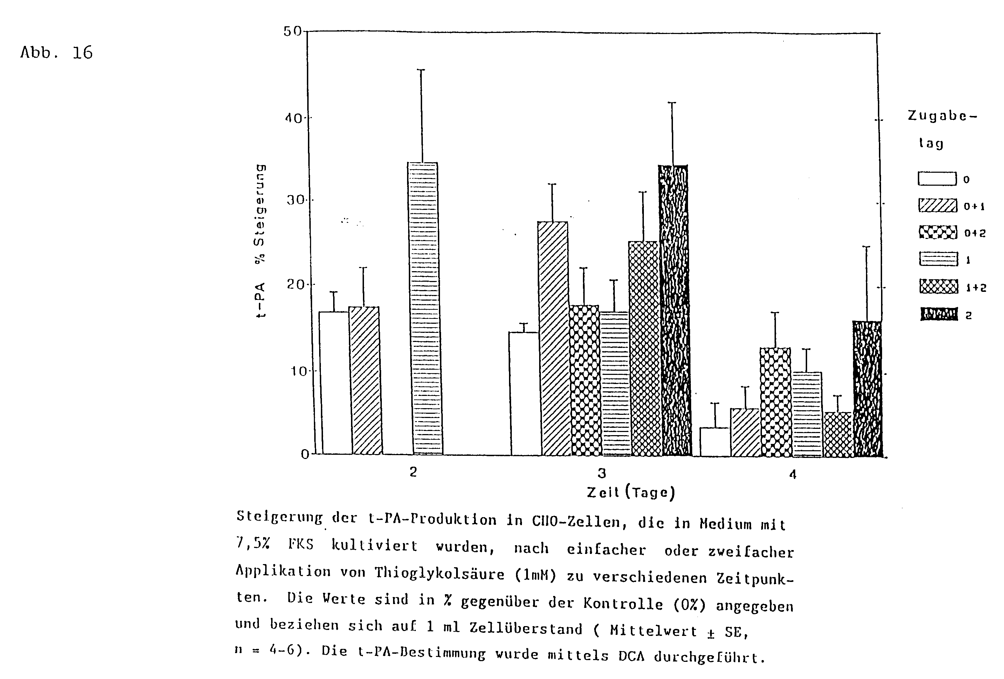

Die rt-PA-Produktion kann durch eine zweifache Applikation von Thioglykolsäure gegenüber der einfachen Applikation erhöht werden (Abb. 16).

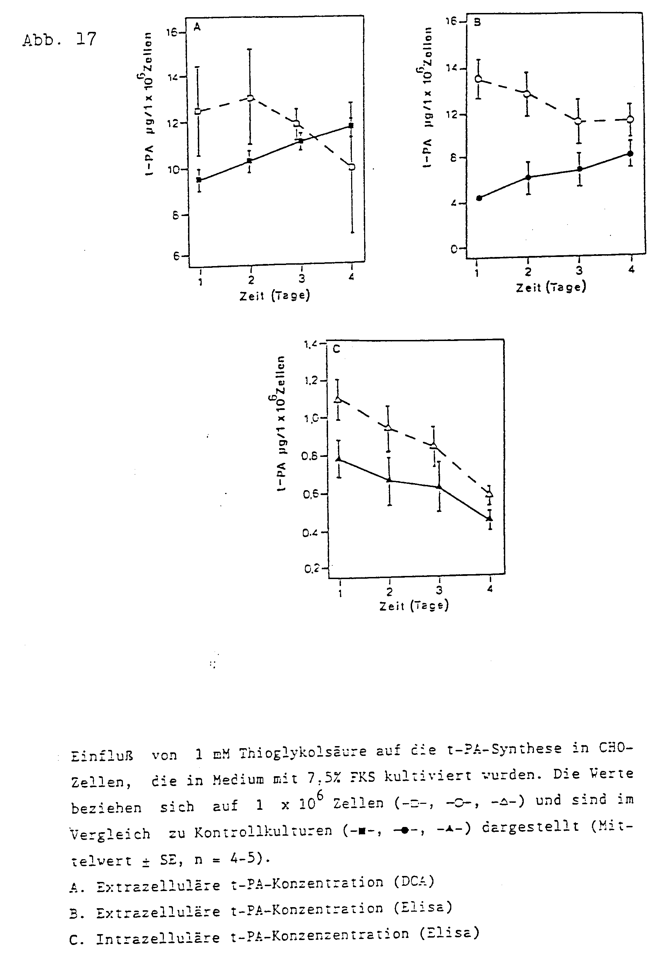

In Abb. 17A (DCA) und Abb. 17B (Elisa) ist der Verlauf der rt-PA-Synthese bezogen auf 1 x 10⁶ Zellen dargestellt. Die rt-PA-Synthese ist durch 1 mM Thioglykolsäure die ersten 3 Tage (DCA) gegenüber der Kontrolle erhöht. Die im Elisa ermittelten rt-PA-Werte bleiben hingegen über den ganzen Zeitraum erhöht. Die Steigerungsrate beträgt am 4. Tag noch ca. 35%. Der intrazelluläre rt-PA-Gehalt pro Zelle wird durch Thioglykolsäure ebenfalls erhöht. Die maximale intrazelluläre rt-PA-Konzentration beträgt 1,1 ± 0,1 »g/1 x 10⁶ Zellen nach 24 h und fällt innerhalb 96 h ungefähr auf die Hälfte der ursprünglichen Konzentration herab (Abb. 17C).

Die extrazelluläre rt-PA-Konzentration ist am 5. Tag 10,7% gegenüber der Kontrolle gesteigert. Die prozentuale Steigerung der rt-PA-Synthese durch Thioglykolsäure ist in serumfreiem Medium etwas geringer, da die Zellteilung leicht gehemmt wird (10-20%).

### Effekte von Derivaten der Monocarbonsäuren auf die rt-PA-Produktion in CHO-Zellen

In der Gruppe der C₄-Carbonsäurederivate wurden weitere Substanzen gefunden, die eine Wirkung auf die rt-PA-Synthese in CHO-Zellen zeigen. Zu diesen Substanzen zählen in erster Linie Butyrylcholinbromid (BCB) und -chlorid (BCC), die die rt-PA-Produktion zwischen 30% und 40% steigern. Diese C₄-Derivate zeigen in jeder Hinsicht die gleichen Effekte wie Na-Butyrat und sind im gleichen Konzentrationsbereich wirksam.

In Tabelle 10 ist die Steigerung der rt-PA-Produktion in CHO-Zellen durch die zwei oben erwähnten Derivate der Monocarbonsäuren dargestellt. Die Zellen wurden in Medium mit 7,5% FKS kultiviert. Alle Substanzen wurden in einem Konzentrationsbereich von 10 »M bis 10 mM getestet. Die Werte sind in % gegenüber der Kontrolle (0%) angegeben und beziehen sich auf 1 ml Zellüberstand (Mittelwert ± SE, n = 4-15). Die Werte, die sich auf die Einzelproben beziehen, sind in Klammern angegeben. Der maximale Wert bezieht sich auf eine Einzelprobe. Die rt-PA-Bestimmung wurde mittels DCA durchgeführt.

**TABELLE 10**

| Derivate der Monocarbon säuren | optimaler Dosisbereich | % Steigerung der rt-PA-Synthese | | n |
|---|---|---|---|---|
| | | Maximum | Durchschnitt | |
| BCC | 5-10 mM ( 10 mM) | 63,9 | 38,8 ± 4,4 | 15 |
| BCB | 5-10 mM (2.5 mM) | 49,6 | 29,1 ± 4,4 | 9 |

Die Durchschnittswerte beziehen sich auf mehrere Produktionsgänge von 4 Tagen wie in Tabelle 10A dargestellt. Werte für die Einzelproben sind in Klammern angegeben.

**TABELLE 10A**

| Substanz | Zugabetag | Probetage | n |
|---|---|---|---|
| BCC | 1 (1) | 2-4 (4) | 15 |
| BCB | 1 (1) | 2-4 (4) | 9 |

### Kombinierte Effekte verschiedener rt-PA-Synthese-Stimulatoren

Bei Verwendung von Substanzkombinationen sollte mindestens eine Substanz die Eigenschaft besitzen, daß sie das Zellwachstum nicht hemmt.

Eine erhöhte Ausbeute wurde z.B. bei einer Kombination von Thioglykolsäure und Buttersäure erreicht. Eine wichtige Rolle kann dabei der Applikationszeitraum der einzelnen Substanzen spielen, wobei z.B. Buttersäure immer als 2. Substanz zugegeben wurde, um die inhibitorische Wirkung auf das Zellwachstum zu verringern.

Bei einer gleichzeitigen Applikation der Substanzen am Tag 0 wird der Effekt von der Buttersäure nicht erhöht.

Für die Kombination Buttersäure und Thioglykolsäure ist der Effekt additiv. Als Steigerungseffekt konnte maximal 70% erreicht werden.

In serumfreiem Medium konnten die Effekte von Buttersäure durch eine weitere Substanz nicht weiter gesteigert werden.

Die Steigerung der rt-PA-Produktion in CHO-Zellen, die 3 Tage in Medium mit 7,5% FKS kultiviert werden ist in Tabelle 11 dargestellt. 1 mM Thioglykolsäure (TG) und 1 mM Buttersäure wurden in Kombination zu verschiedenen Zeitpunkten den Zellen zugesetzt. Die Werte beziehen sich auf 1 ml Zellüberstand und sind in % gegenüber der Kontrolle (0%) angegeben (Mittelwert ± SE, n = 4-8). Die rt-PA-Bestimmung wurde mittels DCA durchgeführt.

**TABELLE 11**

| Applikationstag | | | |
|---|---|---|---|
| TG | Buttersäure | | |
| | 0 | 1 | 2 |
| 0 | 31,3 ± 1,7 | 67,9 ± 5,2 | 30,3 ± 3,2 |
| 1 | | 68,8 ± 4,4 | 31,4 ± 5,5 |
| 2 | | | 41,4 ± 5,5 |

### Effekte von Aphidicolin

Aphidicolin, ein Diterpen aus Cephalosporium aphidicola, hemmt spezifisch die DNA alpha-Polymerase und stimuliert die rt-PA-Synthese in CHO-Zellen in einer Konzentration von 1 bis 10 »M. Nach 24 h ist die rt-PA-Ausbeute um 44,9 ± 13,9% gesteigert.

Aphidicolin zeigte eine Stimulierung der rt-PA-Synthese in CHO-Zellen. Es kann die DNA-Synthese reversibel hemmen, dabei wird die RNA- und Proteinsynthese nicht beeinflußt. Aphidocolin eignet sich also einerseits, die rt-PA-Synthese zu stimulieren und andererseits die Zellen zu synchronisieren. Der Effekt von Aphidicolin auf die rt-PA-Synthese bei längerer Inkubation der Zellen in Medium mit Aphidicolin ist oben beschrieben. Dieser Passus umschreibt Arbeiten mit CHO-Zellen die 24 h dem Aphidicolin ausgesetzt waren und anschließend in Medium ohne Aphidicolin weiterkultiviert wurden. Die Phasen des Zellzyklus und der Einbau von [³H]-Thymidin während der 24 h-Markierung und danach wurden analysiert, um die Hemmung der DNA-Synthese und eine Wiederaufnahme der Synthese nach Entfernen des Aphidicolin zu verfolgen. Weiterhin wurde die RNA-, Protein- und die rt-PA-Synthese bestimmt.

### Zellwachstum

Das Zellwachstum war durch Aphidicolin (1 »M) nach einer 24 h-Behandlung leicht gehemmt (5-10%). Beim Mediumwechsel wurden die Aphidicolin-behandelten Kulturen jedoch wieder auf die gleiche Zellzahl wie die Kontrollkulturen gesetzt.

### rt-PA-Synthese

Die rt-PA-Synthese war in 1 »M Aphidicolin-synchronisierten Zellen stimuliert. Ein Effekt war noch 96 h nach Mediumwechsel zu sehen.

Die Steigerung der rt-PA Produktion in 1 »M Aphidicolin-behandelten CHO-Zellen, die in Medium mit 7.5% FKS kultiviert wurden, ist in Tabelle 12 angegeben. Die Werte beziehen sich auf 1 ml Zellüberstand und sind in % gegenüber der Kontrolle (0%) angegeben (Mittelwert ± SE, n = 4). Die rt-PA-Bestimmung wurde mittels DCA durchgeführt.

**TABELLE 12**

| Zeit (h nach Mediumwechsel) | % Steigerung der rt-PA-Synthese |
|---|---|
| 24 | 40,2 ± 6,7 |
| 48 | 59,9 ± 11,1 |
| 72 | 32,8 ± 6,4 |
| 96 | 14,7 ± 1,3 |

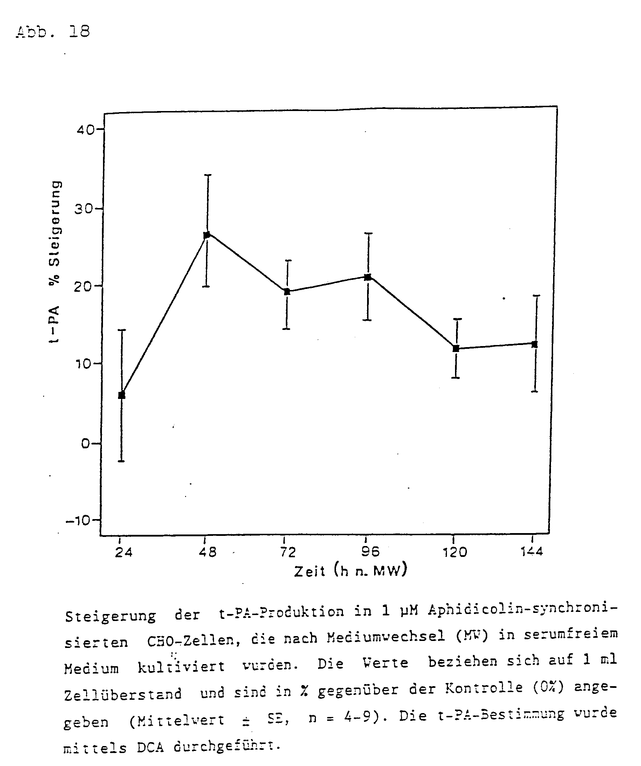

In Abb. 18 ist der zeitliche Verlauf der rt-PA-Synthesesteigerung in serumfreiem Medium dargestellt. Der Effekt erreicht ein Maximum 48 h nach Mediumwechsel.

Die rt-PA-Konzentration in serumfreiem Medium wurde nach 96, 120 und 144 h auch mittels Elisa bestimmt, die gegenüber den Kontrollwerten wesentlich höher lagen als die im DCA ermittelten Werte. Die Ergebnisse sind in Tabelle 13 angegeben. Der Test wurde mit 1 »M Aphidicolin-behandelten CHO-Zellen, die in serumfreiem Medium kultiviert wurden, durchgeführt. Die Werte beziehen sich auf 1 ml Zellüberstand und sind in % gegenüber der Kontrolle (0%) angegeben (Mittelwert ± SE, n = 4). Die rt-PA-Bestimmung wurde mittels Elisa durchgeführt.

**TABELLE 13**

| Zeit (h nach Mediumwechsel) | % Steigerung der rt-PA-Synthese |
|---|---|
| 96 | 36,7 ± 6,2 |
| 120 | 42,0 ± 8,8 |
| 144 | 51,3 ± 10,2 |

Eine erhöhte intrazelluläre rt-PA-Konzentration konnte nur während der 24 h-Inkubation mit Aphidicolin beobachtet werden und war ca. 30% erhöht.

Buttersäure, Propionsäure, Butyrylcholinchlorid und -bromid konnten in diesem System die rt-PA-Produktion noch weiter steigern. Die Stimulierung war sowohl in serumfreiem als auch in serumreichem Medium erfolgreich, wobei ein zusätzlicher Steigerungseffekt in serumfreiem Medium nur bei Applikation der Substanzen nach einer 24 h-Anpassung der Zellen an diese Mediumbedingung erreicht wurde.

Tabelle 14 zeigt die prozentuale Steigerung der rt-PA-Produktion in Aphidicolin-behandelten Kulturen nach Applikation von Na-Butyrat. Na-Butyrat steigert die rt-PA-Synthese um weitere 14% nach 96 h.

Der Test wurde mit 1 »M Aphidicolin-behandelten CHO-Zellen, die mit und ohne Na-Butyrat kultiviert wurden, in serumfreier Kultur durchgeführt. Die Werte beziehen sich auf 1 ml Zellüberstand und sind in % gegenüber der Kontrolle (0%) angegeben (Mittelwert ± SE, n = 4). Die rt-PA-Bestimmung wurde mittels Elisa durchgeführt.

**TABELLE 14**

| Zeit (h nach Mediumwechsel) | Aphidicolin | + Na-Butyrat Applikationszeitpunkt (h) | |
|---|---|---|---|
| | | 0 | 24 |
| 96 | 36,7 ± 6,2 | 34,1 ± 9,7 | 50,6 ± 11,0 |
| 120 | 42,0 ± 8,8 | 31,8 ± 10,2 | 49,7 ± 4,3 |

### Effekte von 6-Hydroxy-4,6-dimethyl-3-hepten-2-on (DHO)

Die rt-PA Produktion ist nach der Behandlung mit der obigen Verbindung nach 144 h erhöht. Für die Stimulierung der rt-PA-Synthese waren Konzentrationen im micromolar Bereich wirksam z.B. 0,005 bis 100 »M.

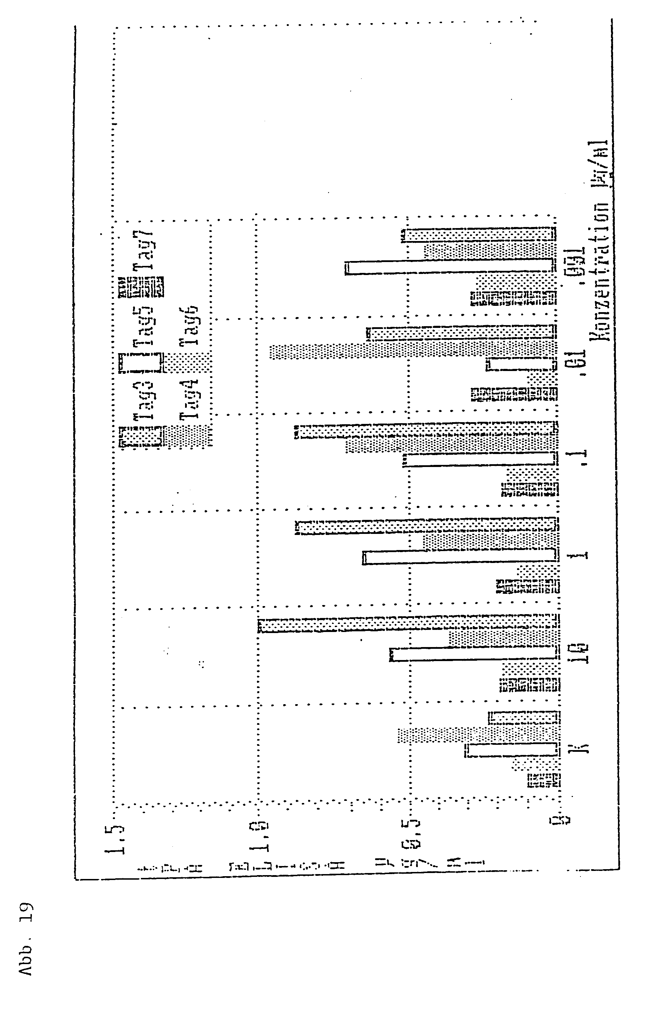

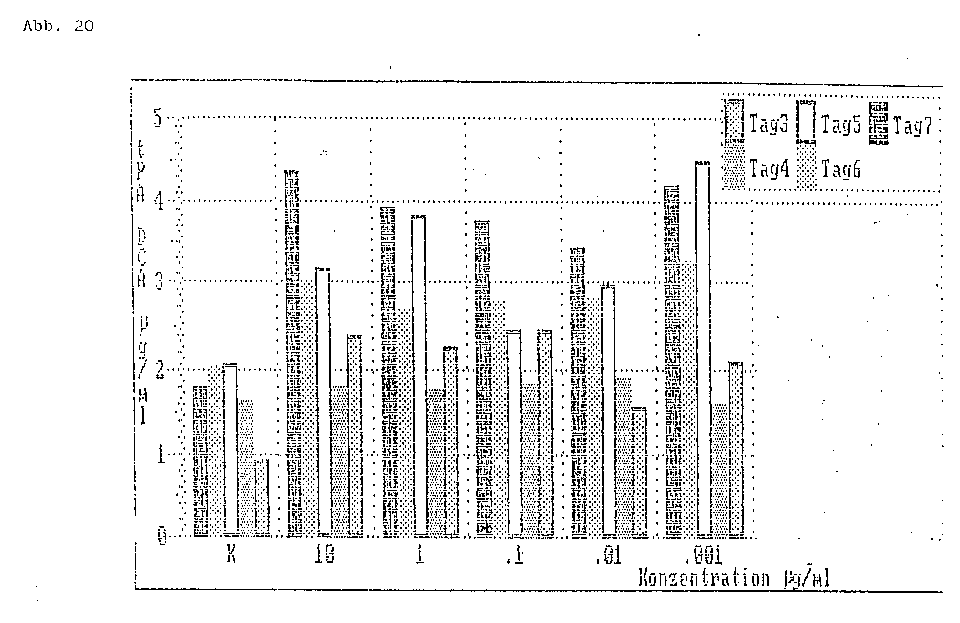

In Abb. 19 ist die Steigerung der rt-PA Produktion in CHO-Zellen, die bis zu 168 h in serumfreiem Medium und 7 nM bis 7 »M DHO kultiviert wurden, dargestellt. Die Werte wurden mittels DCA durchgeführt. In Abb. 20 sind die entsprechenden Werte, die mittels Elisa durchgeführt wurden, angegeben.

Zellkulturen, die durch die oben beschriebene Methode hergestellt wurden, können in bekannter Weise umgearbeitet werden, um das rt-PA zu isolieren, z.B. nach der Produktionsphase wird das Kulturmedium von der Zellmasse abgetrennt und der Zellüberstand durch Ultrafiltration und chromatographische Schritte gereinigt.

Das isolierte rt-PA kann danach in pharmazeutische Präparate formuliert werden, z.B. als Lösung oder Lyophilisat.

Man kann die obigen Beispiele unter Austausch der rt-PA produzierten CHO-Zellen durch transformierte CHO-Zellen, die andere Proteine exponieren, z.B. rt-PA-Mutanten oder andere pharmakologisch wirksame Proteine, wiederholen, wie z.B. in den oben genannten Europäischen und Deutschen Patentanmeldungen, insbesondere die CHO-Zellen in EP-A-0,199,574 und 0,196,920 sowie in DE-A-3,708,681.

## Patentansprüche

1. Methode zur Steigerung der Produktion von einem Protein aus Kulturen von transformierten CHO-Zellen, dadurch gekennzeichnet, daß man eine C₃-C₄ aliphatische Monocarbonsäure oder deren Salz der Kultur zusetzt und die Kultivierung in serumfreiem Medium durchführt.

2. Methode gemäß Anspruch 1, dadurch gekennzeichnet, daß das Protein rt-PA oder ein Mutant davon ist.

3. Methode gemäß Anspruch 1 oder 2, dadurch gekennzeichnet, daß man als Protein-induzierende Substanz Buttersäure oder deren Salz verwendet.

4. Methode gemäß Anspruch 1 bis 3, dadurch gekennzeichnet, daß man die C₃-C₄ aliphatische Monocarbonsäure oder deren Salz in einer Konzentration von 1 mM bis 10 mM zusetzt.

5. Methode gemäß einem der Ansprüche 1 bis 4, dadurch gekennzeichnet, daß man die Zellen in Anwesenheit einer der Substanzen ausgewählt von Thioglykolsäure, Thiodiglykolsäure, L-Cystein, Glutathion, Butyrylcholinbromid, Butyrylcholinchlorid, Nonactinsäure, Furanfettsäure, Ascorbinsäure, Aphidicolin, 6-Hydroxy-4,6-dimethyl-3-hepten-2-on, D-α-hydroxysubstituierte (C₃ oder C₄) aliphatische Mono- oder Dicarbonsäure oder deren Salze kultiviert,
die Zellen von der Kultur entfernt und danach in neues serumfreies Kultivierungsmedium gibt und in Anwesenheit von einer C₃-C₄ aliphatischen Monocarbonsäure oder deren Salze kultiviert.

6. Methode gemäß Anspruch 5, dadurch gekennzeichnet, daß Aphidicolin die zuerst erwähnte Substanz ist und die C₃-C₄ aliphatische Monocarbonsäure Buttersäure, Propionsäure oder deren Salze ist.

7. Methode gemäß Anspruch 5, dadurch gekennzeichnet, daß man Buttersäure und Thioglykolsäure oder deren Salze verwendet.

## Claims

1. Method of increasing the production of a protein from cultures of transformed CHO cells, characterised in that a C₃-C₄ aliphatic monocarboxylic acid or a salt thereof is added to the culture, and cultivation is carried out in serum-free medium.

2. Method according to claim 1, characterised in that the protein is rt-PA or a mutant thereof.

3. Method according to claim 1 or 2, characterised in that butyric acid or a salt thereof is used as the protein-inducing substance.

4. Method according to claims 1 to 3, characterised in that the C₃₋₄ aliphatic monocarboxylic acid or a salt thereof is added in a concentration of from 1 mM to 10 mM.

5. Method according to one of claims 1 to 4, characterised in that the cells are cultivated in the presence of a substance selected from thioglycolic acid, thiodiglycolic acid, L-cysteine, glutathione, butyryl choline bromide, butyryl choline chloride, nonactic acid, furan fatty acid, ascorbic acid, aphidicolin, 6-hydroxy-4,6-dimethyl-3-hepten-2-one, D-α-hydroxysubstituted (C₃ or C₄) aliphatic mono- or dicarboxylic acid or the salts thereof, the cells are removed from the culture and then placed in new serum-free culture medium and cultivated in the presence of a C₃₋₄ aliphatic monocarboxylic acid or a salt thereof.

6. Method according to claim 5, characterised in that aphidicolin is the first-mentioned substance and the C₃₋₄ aliphatic monocarboxylic acid is butyric acid, propionic acid or the salts thereof.

7. Method according to claim 5, characterised in that butyric acid and thioglycolic acid or the salts thereof are used.

## Revendications

1. Procédé pour augmenter la production d'une protéine à partir de cultures de cellules CHO transformées, caractérisé en ce que l'on ajoute à la culture un monoacide carboxylique aliphatique en C₃-C₄ ou son sel et on réalise la culture dans un milieu exempt de sérum.

2. Procédé selon la revendication 1, caractérisé en ce que la protéine est le rt-PA ou un mutant de celui-ci.

3. Procédé selon la revendication 1 ou 2, caractérisé en ce que l'on utilise comme substance inductrice de protéine l'acide butyrique ou son sel.

4. Procédé selon les revendications 1 à 3, caractérisé en ce que l'on ajoute le monoacide carboxylique aliphatique en C₃-C₄ ou son sel en une concentration de 1 mM à 10 mM.

5. Procédé selon l'une des revendications 1 à 4, caractérisé en ce que l'on cultive les cellules en présence de l'une des substances choisies parmi l'acide thioglycolique, l'acide thiodiglycolique, la L-cystéine, le glutathion, le bromure de butyrylcholine, le chlorure de butyrylcholine, l'acide nonactique, un acide gras furanique, l'acide ascorbique, l'aphidicoline, la 6-hydroxy-4,6-diméthyl-3-heptén-2-one, un mono- ou diacide carboxylique aliphatique (en C₃ ou C₄) D-α-hydroxysubstitué ou de ses sels, on retire les cellules de la culture puis on les place dans un nouveau milieu de culture exempt de sérum et on les cultive en présence d'un monoacide carboxylique aliphatique en C₃-C₄ ou de ses sels.

6. Procédé selon la revendication 5, caractérisé en ce que l'aphidicoline est la substance citée en premier lieu et le monoacide carboxylique aliphatique en C₃-C₄ est l'acide butyrique, l'acide propionique ou leurs sels

7. Procédé selon la revendication 5, caractérisé en ce que l'on utilise l'acide butyrique et l'acide thioglycolique ou leurs sels.
